# EUROPEAN PATENT APPLICATION

(11) **EP 2 527 836 A1**
(43) Date of publication of application: **28.11.2012**
(21) Application number: 11004320.5
(22) Date of filing: 25.05.2011
(51) Int. Cl.: G01N 33/50

(54) **Novel bone marrow components and methods of their use**

(71) Applicant: Max-Planck-Gesellschaft zur Förderung der Wissenschaften e.V., 80539 München (DE)
(72) Inventor: Adams, Ralf, 48163 Münster (DE); Wang, Lin, 48161 Münster (DE); Kiefer, Friedemann, 48159 Münster (DE)
(74) Representative: Vossius & Partner

(57) **Abstract**

The present invention relates to a bone marrow component comprising at least one capillary embedded in a bone culture wherein the capillary comprises (i) an inner cell layer of endothelial cells surrounding an inner lumen that is connected to the vasculature, and (ii) an outer cell layer comprising mesenchymal cells, osteoblasts, and osteoclasts; and wherein at least part of the distal end of capillary is dilated compared to the proximal end and at least part of the dilated area has an outer cell layer that is physically separated from the inner cell layer. The present invention further relates to a method of monitoring bone marrow status of a subject comprising detecting at least one bone marrow component of the invention within a bone marrow sample provided from a subject.

## Description

The present invention relates to a bone marrow component comprising at least one capillary embedded in a bone culture wherein the capillary comprises (i) an inner cell layer of endothelial cells surrounding an inner lumen that is connected to the vasculature, and (ii) an outer cell layer comprising mesenchymal cells, osteoblasts, and osteoclasts; and wherein at least part of the distal end of capillary is dilated compared to the proximal end and at least part of the dilated area has an outer cell layer that is physically separated from the inner cell layer. The present invention further relates to a method of monitoring bone marrow status of a subject comprising detecting at least one bone marrow component of the invention within a bone marrow sample provided from a subject.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

One of the most fundamental questions in stem cell biology is how stem cells, which were first generated during embryonic or fetal life, are maintained in the adult organism. The stem cell niche theory implies that a local, specialized microenvironment (i.e., niche) controls stem cell behavior presumably through defined cell-cell and cell-matrix interactions in combination with secreted factors (Li and Xie, 2005; Moore and Lemischka, 2006; Morrison and Spradling, 2008; Scadden, 2006). While the hematopoietic system has been studied extensively as a paradigm for understanding the developmental hierarchy, self-renewal and differentiation of somatic stem cells (Adams and Scadden, 2006), the nature of the hematopoietic niche remains enigmatic. Critical roles have been attributed to a variety of cell types. Endosteal osteoblasts, which line the inner surface of long bones, can regulate hematopoietic stem cell (HSC) development in the bone marrow (BM) (Calvi et al., 2003; Zhang et al., 2003). CXCL12-abundant reticular (CAR) cells, which are found in the endosteum (a layer of connective tissue lining the BM cavity) but also in close association with sinusoidal endothelial cells (SECs), are required for the maintenance of the HSC pool (Sugiyama et al., 2006). CD146-positive osteoprogenitors, the mesenchymal precursors that give rise to osteoblasts, together with sinusoidal endothelial cells (SECs) can organize an ecotopic hematopoietic niche after xenotranplantation (Sacchetti et al., 2007). Nestin-expressing mesenchymal stem cells form a niche with HSCs that is influenced by the surrounding microenvironment and hormonal signals (Mendez-Ferrer et al., 2010). Arguing for a role of SECs in constructing functional hematopoietic niches, CD150+/CD41-/CD48- HSCs physically interact with sinusoidal capillaries in both BM and, in certain settings, the spleen, which does not contain osteoblasts (Kiel et al., 2005). Furthermore, recent work has suggested that direct signaling interactions between SECs and hemotopoietic stem cells through the Notch pathway are involved in the self-renewal of HSCs and their capacity to repopulate BM in vivo (Kiel et al., 2005).

Notch signaling, which controls cell proliferation, differentiation and patterning processes in a wide range of tissues and organisms, is also known to regulate the angiogenic growth of blood vessels, a role that is coupled to the activity of the vascular endothelial growth factor (VEGF) pathway (Benedito et al., 2009; Hellstrom et al., 2007; Siekmann and Lawson, 2007). In support of a role for angiogenic signaling in the sinusoidal endothelium, inhibition of the VEGF receptor VEGFR2/Flk1 was found to impair osteogenesis and the repopulation of bone marrow by transplanted HSCs in lethally irradiated animals (Hooper et al., 2009). Conversely, inducible over-expression of VEGF in the adult bone resulted in increased angiogenesis and bone formation as well as alterations in hematopoiesis (Maes et al., 2010).

Despite this recent progress, the precise interactions between the cellular components of the hematopoietic niche, its organization in space and time, and the mechanisms underlying bone marrow formation and homeostasis remain largely unclear. Consequently, there is still a need to identify the physiological origins of the bone marrow as well as a need for information and understanding about bone marrow development, morphology and homeostasis, also because of the implication of the bone marrow components in disease processes.

These needs are addressed by the provision of the embodiments characterized in the claims.

Accordingly, the present invention relates in a first embodiment to a bone marrow component comprising at least one capillary embedded in a bone scaffold or bone culture wherein the capillary comprises (i) an inner cell layer comprising endothelial cells surrounding an inner lumen that is connected to the vasculature, and (ii) an outer cell layer comprising mesenchymal cells, osteoblasts, and osteoclasts; and wherein the distal end of capillary is dilated compared to the proximal end and at least part of the dilated area has an outer cell layer that is physically separated from the inner cell layer.

The bone marrow component of the present invention relates to the specialized environment within the bone marrow that controls bone marrow formation, hematopoietic stem cell maintenance and hematopoiesis. Mesenchymal stem cells are known to promote HSC maintenance (Mendez-Ferrer et al., 2010), and the bone marrow components contain stem cells, LT-HSC's, and progenitor cells. As is also shown herein, the interactions therein between endothelial cell layers and mesenchymal cells/cell layers are directly involved in the recruitment and maintenance of stem cells into the bone marrow components. The manipulation of signaling pathways in the endothelial compartment directly affects perivascular cell types and also affects the abundance of mesenchymal stem cells and osteoprogenitor cells. The invention also demonstrates that manipulation of angiogenic pathways and endothelial-derived signals affects the numbers of mesenchymal cells, numbers of mesenchymal stem cells, bone marrow formation, numbers of hematopoietic stem cells (HSCs) and the functional characteristics of HSCs. Thus the bone marrow component of the present invention represents the earliest state, or the beginning, of formation of the bone marrow and the hematopoietic system in a subject, including the ongoing renewal of the bone marrow. As such, the bone marrow component of the invention is an ideal indicator of the bone marrow status. Detection of changes in the bone marrow component can provide the earliest and most accurate indications of changes, both positive and negative, in the bone marrow. This early detection can occur before such changes are detectable within the hematopoietic system and/or circulating blood of the subject, or before such changes cause adverse effects within the hematopoietic system of the subject.

Physically separated, as used herein, relates to a lack of structural connections between the cells or cell layers. Thus, for example, two cells or cell layers can be physically separated such that the distance between them is easily detectable or they can be in very close physical proximity to each other and still be physically separated (*i.e*., the cells or cell layers have no physical connections between them such as proteins or carbohydrate chains that are physically binding to each cell or cell layer).

The morphology of the bone marrow component of the present invention, also described herein as a "hemosphere," is made up of at least one capillary. The inner cell layer of the capillary comprises endothelial cells which form a continuous layer surrounding an inner lumen. Covering this inner layer of endothelial cells is an outer layer that includes, but is not limited to mesenchymal cells, osteoblasts and osteoclasts.

The capillary of the bone marrow component is also dilated at the distal end, compared to the diameter or circumference of the proximal end of the capillary. The proximal end of the capillary encompasses the stalk of the capillary and leads away from the dilated distal end (i.e., the bulge), and generally leads towards the established lacuna of the bone marrow. Dilated, distal ends can also extend away from the proximal end at positions along the proximal end/capillary stalk. Thus dilated, distal ends can occur as multiple branches off the proximal end of the capillary, with each branch having a dilated distal end. Thus, the dilated distal end relates to dilation of the entire distal end of the capillary and/or dilation of at least part of the distal end of the capillary. Dilation of at least part of the distal end of a capillary also includes, for example, the dilation of a small area, segment or portion of the capillary distal end, or a bulge in an area, segment or portion of the distal end of a capillary. This dilation of at least part of the distal end of the capillary can also be described as initiation of bone marrow component development or hemosphere initiation. The dilated area of at least part of the distal end of the capillary can enlarge as the bone marrow component increases in size and diameter. Thus a bone marrow component can have dilated areas that encompass part of the distal end of the capillary, the entire distal end of the capillary, and any amount of the distal capillary end in between. Additionally, at a dilated, distal end, at least part of the outer cell layer of the capillary is physically separated from the inner cell layer, resulting in a further dilation of the distal end. As more of the outer cell layer of the capillary separates from the inner cell layer, the diameter grows larger and the physical space between the outer cell layer and the inner cell layer also grows larger. This growth can also be described as bone marrow component expansion, or hemosphere expansion.

Within the dilated capillary, the inner cell layer surrounding the inner lumen, also described herein as the central vessel of the bone marrow structure, is also dilated. This central vessel can have a variety of shapes, such as a cone-shaped structure or a bulbous shaped structure, with a diameter of 5 µm or less, 10 µm or less, 15 µm or less, 20 µm or less, 25 µm or less, 30 µm or less, 35 µm or less, 40 µm or less, 45 µm or less, 50 µm or less, 55 µm or less, 60 µm or less, 65 µm or less, 70 µm or less, 75 µm or less, 80 µm or less, 85 µm or less, 90 µm or less, 95 µm or less, 100 µm or less, 105 µm or less, 110 µm or less, 115 µm or less, 120 µm or less, 125 µm or less, 130 µm or less, 135 µm or less, 140 µm or less, 145 µm or less, 150 µm or less, 155 µm or less, 160 µm or less, 165 µm or less, 170 µm or less, 175 µm or less, 180 µm or less, 185 µm or less, 190 µm or less, 195 µm or less, 200 µm or less, 205 µm or less, 210 µm or less, 215 µm or less, 220 µm or less, 225 µm or less, 230 µm or less, 235 µm or less, 240 µm or less, 245 µm or less, and 250 µm or less. The diameter values further include all values in between each specified value such. The inner diameters can also be represented as falling within ranges, where the upper and lower ends of the ranges comprise any of the identified diameters in combination with each other. Because it is located within the outer cell layer, the inner lumen is necessarily of a smaller diameter than the outer diameter formed by the outer cell layer. Thus, while the disclosed values and ranges of the inner diameter and outer diameter overlap, it is understood that the outer diameter is a value that encompasses the inner diameter.

The dilated, distal end of the capillary encompasses varying sizes and has varying outer diameters depending upon the extent or amount of separation of the outer cell layer from the inner cell layer. The outer diameter can have diameters of, for example, 10 µm or less, 15 µm or less, 20 µm or less, 25 µm or less, 30 µm or less, 35 µm or less, 40 µm or less, 45 µm or less, 50 µm or less, 55 µm or less, 60 µm or less, 65 µm or less, 70 µm or less, 75 µm or less, 80 µm or less, 85 µm or less, 90 µm or less, 95 µm or less, 100 µm or less, 105 µm or less, 110 µm or less, 115 µm or less, 120 µm or less, 125 µm or less, 130 µm or less, 135 µm or less, 140 µm or less, 145 µm or less, 150 µm or less, 155 µm or less, 160 µm or less, 165 µm or less, 170 µm or less, 175 µm or less, 180 µm or less, 185 µm or less, 190 µm or less, 195 µm or less, 200 µm or less, 205 µm or less, 210 µm or less, 215 µm or less, 220 µm or less, 225 µm or less, 230 µm or less, 235 µm or less, 240 µm or less, 245 µm or less, 250 µm or less, 255 µm or less, 260 µm or less, 265 µm or less, 270 µm or less, and 275 µm or less, including all values in between. The outer diameters can also be represented as falling within ranges, where the upper and lower ends of the ranges comprise any of the previous diameters in combination with each other.

Based on the features described above, the bone marrow component of the present invention can be found in different mammalian species (e.g., mice, humans, etc.) and exists in a range of skeletal elements, including long bone, the sternum and the skull. The bone marrow component is dynamic and can vary in size. For example, a small bone marrow component can have a minimum outer diameter of 5 µm whereas the diameter increases during the development of the bone marrow components. Additional examples of outer diameter ranges for a bone marrow component include, but are not limited to, 5 µm to 275 µm or less, a preferred range of 20 µm to 250 µm or less, and a more preferred range of 30 µm to 150 µm or less, including all values within each range. The number of hematopoietic cells found in the space formed between the inner endothelial layer and the outer mesenchymal layer also varies among the different sizes of a bone marrow component. Finally, the enlargement of a bone marrow component can result in fusion between two or more bone marrow components when they physically meet due to the enlargement of their diameters. This can be described as bone marrow component fusion or hemosphere fusion. This fusion process eventually generates lacuna that becomes the established bone marrow cavity. This process is believed to be continuous, thus the bone marrow component continuously replenishes or creates additional mature bone marrow. The bone marrow component of the present invention is the previously unknown precursor to the established bone marrow.

As used herein, the term "endothelial cell" is well-known by one of skill in the art and relates to cells that line the interior surface of blood vessels, forming an interface between circulating blood in the lumen and the rest of the vessel wall. Endothelial cells line the vessels of the entire circulatory system. Endothelial cells include, but are not limited to, continuous endothelial cells, fenestrated endothelial cells, and discontinuous endothelial cells. Continuous endothelial cells are tightly connected together by a series of tight junctions and gaps. They line major blood vessels and capillaries. Fenestrated endothelial cell junctions are similar to those of continuous endothelium, but they contain fenestrae, or "windows". These are openings in the plasma membrane between the inside of the capillary and the tissue beneath it. Fenestrated endothelial cells can be found, although not exclusively, in the gastrointestinal villi, glomeruli of the kidneys, and capillaries that supply the endocrine glands Discontinuous endothelial cells are not connected to each other, allowing for a different type of cell to be interspersed with the endothelial cells or the underlying cellular matrix may be exposed to the inner part of the capillary. The capillaries with discontinuous endothelial cells are called sinusoidal capillaries. This type of endothelium occurs, although not exclusively, in bone marrow, the spleen and the liver.

Mesenchymal cells, as described herein, are known to one of skill in the art and encompass cells capable of developing into connective tissues, such as bone, cartilage or adipose tissue. Mesenchymal stem cells, in accordance with the present invention, relate to multipotent stem cells that can differentiate into a variety of cell types during *in vitro* culture, including: osteoblasts and osteoclasts (bone cells), chondrocytes (cartilage cells) and adipocytes (fat cells). The standard test to confirm multipotency is differentiation of the cells into osteoblasts/osteoclasts, adipocytes, and chondrocytes as well as myocytes and possibly neuron-like cells. Furthermore, the cell surface determinants CD146 and Nestin are also understood within the art as markers of mesenchymal stem cells. Additional markers and techniques for the identification of mesenchymal stem cells are known to one of skill in the art and contemplated herein.

Osteoclasts and osteoblasts, as described herein, are also known to one of skill in the art and relate to differentiated mesenchymal cells. Osteoclasts further relate to cells that remove bone tissue by removing its mineralized matrix and breaking up the organic bone. Exemplary osteoclast markers include, but are not limited to, CD14, CD33, vitronectin receptor (VR), calcitonin receptor, tartrate resistant acid phosphatase (TRAP), and cathepsin K protein. Osteoblasts further relate to cells that are responsible for bone formation by producing osteoid and mineralizing the osteoid matrix. Examples of osteoblast markers include, but are not limited to, Runx2, osterix, N-cadherin, and osteopontin. Additional markers for identification of osteoblasts and osteoclasts are known in the art and contemplated herein.

In a preferred embodiment, the bone marrow component of the present invention comprises at least one hematopoietic cell located between the inner cell layer and the outer cell layer at the distal end of the capillary.

Hematopoietic cells, in accordance with the present invention and as used herein, are understood by one of skill in the art and encompass hematopoietic cells, hematopoietic stem cells (HSCs), and long-term hematopoietic stem cells (LT-HSCs). Hematopoietic stem cells (HSCs), in accordance with the present invention, relate to multipotent stem cells that give rise to all the hematopoietic cell types including, but not limited to, myeloid cells (thrombocytes, monocytes and macrophages, neutrophils, basophils, eosinophils, erythrocytes, megakaryocytes/platelets, dendritic cells) and the lymphoid cells (B-cells, T-cells, NK-cells).The markers for each type of hematopoietic cell are known in the art, and identification of the different types of hematopoietic cells can be accomplished through a variety of techniques including, but not limited to, electron microscopy, flow cytometry, immunohistochemistry techniques, Wright-Giemsa staining, and/or laser-scanning confocal microscopy. In accordance with the invention described herein, hematopoietic cells and hematopoietic stem cells reside within the bone marrow component of the present invention.

In a preferred embodiment of the present invention, bone culture comprises bone cultures as well as bone scaffolds. *Ex vivo* bone cultures and bone scaffolds are known in the art and these techniques can be used for the *ex vivo* culture of the bone marrow component of the present invention. Similarly, whole bone culture techniques are well-known in the art and are contemplated herein. Additionally, three-dimensional bone cultures are known in the art for assessment of biological effects of living bone (see Davies, CM et al., European Cells and Materials, Vol. 11: 57-75 (2006); Yeatts and Fisher, Bone 48:171-81 (2011); Davies et al., European Cells and Materials Vol. 11:57-75 (2006)). Bone cultures and bone scaffolds can comprise natural and non-natural materials, native and non-native materials. Non-native and non-natural materials as used herein relate to bone cultures or bone scaffold cultures that include materials that are not derived directly from natural bone and/or are used in combination with natural bone. Such culture methods are also known in the art and can also be used for the ex *vivo* culture of the bone marrow component of the present invention (Song, K. et al., J. Biomed Mater Res A, Aug; 86(2): 323-32 (2008)).

Because the bone marrow component of the present invention represents the earliest state, or the beginning, of formation of the bone marrow and the hematopoietic system in a subject, including the ongoing renewal of the bone marrow, it is an ideal indicator of the bone marrow status. Detection of changes in the bone marrow component can provide the earliest and most accurate indications of changes, both positive and negative, in the bone marrow. This early detection can occur before such changes are detectable within the hematopoietic system and/or circulating blood of the subject, or before such changes cause adverse effects within the hematopoietic system of the subject. Based on the new findings of the bone marrow components of the present invention, novel methods for monitoring bone marrow status, identifying compounds useful for the treatment of cancer, identifying compounds useful for modulating angiogenesis, identifying whether a compound has adverse side effects on the bone marrow, and identifying compounds that promote trabecular bone formation have been developed.

Because the bone marrow component of the present invention reflects a hematopoietic niche within the bone marrow, detection of the bone marrow component is a novel and more precise method of monitoring the status of the bone marrow of a subject. The present invention also relates to a method of monitoring bone marrow status of a subject comprising detecting at least one bone marrow component within a bone marrow sample provided from a subject.

Maintenance of the bone marrow is important to the health of a subject. For example, some therapeutic treatments (e.g., chemotherapy, bone marrow transplantation, etc.) and some diseases (immunosuppressive diseases) can have negative or unwanted effects on the bone marrow. Thus the identification of compounds which modulate bone marrow status would be beneficial. In another embodiment, the present invention relates to a method for identifying a compound that modulates bone marrow status by detecting a bone marrow component within a bone marrow sample provided from a subject that has been previously administered a compound, wherein a change in the morphology of a bone marrow component indicates that the compound modulates bone marrow homeostasis.

Modulate, as used herein, relates to causing changes that are detectable, including both positive and negative changes. Modulation can include, for example, increases or decreases in numbers and/or frequency of the bone marrow component, changes in morphology of the bone marrow component, changes in hematopoiesis, or changes in the number or frequency of HSCs/LT-HSCs. Methods of detecting the changes that reflect modulation in any of the embodiments are described herein and known in the art.

Angiogenesis is involved in a variety of physiological conditions including, but not limited to, wound healing, tissue regeneration, organ development and regeneration, and a variety of diseases having excessive angiogenesis as a causal factor. Because the bone marrow component responds to angiogenic signals, as described herein, it can be useful for identifying compounds which modulate angiogenesis.

In a further embodiment, the present invention relates to a method for identifying a compound that modulates angiogenesis by detecting a bone marrow component within a bone marrow sample provided from a subject that has been previously administered a compound, wherein a change in the morphology of the bone marrow component indicates that the compound modulates bone marrow angiogenesis.

Angiogenesis, as used herein, relates to the physiological process involving the growth of all types blood vessels and includes all types of vessel growth known in the art. Various proteins and factors are known to be involved in angiogenesis, for example, such as signaling via VEGF receptors and Notch receptors.

In an additional embodiment, the present invention also relates to a method for identifying a compound that inhibits cancer by detecting a bone marrow component within a bone marrow sample provided from a subject that has been previously administered a compound, wherein a reduction of the bone marrow components or a change in the bone marrow component morphology indicates that the compound inhibits cancer.

Cancer cells require oxygen and nutrients, and the development of blood vessels is an essential step in the growth of a tumor. Without vessels, tumors cannot grow to be larger than a small fraction of a centimeter. When the area around the cells in a tumor starts to get too far from a blood vessel, the oxygen and nutrient levels start to go down resulting in hypoxia. Hypoxia triggers changes in the behavior of the tumor cells, and the tumor cells produce (or cause nearby cells to produce) growth factors that stimulate the formation of blood vessels. One of the most well-studied angiogenesis factors is called vascular endothelial derived growth factor (VEGF), and the role of angiogenesis factors in cancer development and maintenance is well documented. VEGF, or other angiogenesis factors produced by tumor cells or nearby cells, causes the development of blood vessels that promote the growth of the tumor, and numerous cancer therapeutics are available that target angiogenic ligand receptors, angiogenic ligands, or both to stop the vascularization of the cancer and thus inhibit the cancer.

The bone marrow component of the present invention comprises unique capillaries (i.e., a vascular component) and is directly responsive to angiogenic ligands as described herein. Thus, methods of detecting compounds that affect the bone marrow component, and more specifically inhibit the bone marrow component, are useful for the detecting compounds that can inhibit cancer growth.

In another embodiment, the present invention relates to a method for identifying whether a compound causes adverse side effects on bone marrow homeostasis or hematopoiesis by detecting a bone marrow component within a bone marrow sample provided from a subject that has been previously administered a compound, wherein an inhibition of the bone marrow components or a change in the bone marrow component morphology indicates that the compound causes adverse side effects on bone marrow status or hematopoiesis. In additional preferred embodiments, detecting changes in bone marrow component morphology, bone marrow homeostasis, or hematopoiesis comprise detecting whether a reduction in HSC, LT-HSC or hematopoietic cells associated with the bone marrow component, whether a reduction in capillary endothelial cell growth or whether a reduction of the dilation of capillaries has occurred.

Hematopoiesis as used herein and understood in the art relates to the dynamic developmental process of the formation of hematopoietic cells. These cells include new blood cells and blood components, including all bone marrow-derived cell types in the blood, proteins, factors and components such as, for example, platelets. It further includes mature cell types and their immature precursors that are identifiable by a variety of methods known in the art such as, but not limited to, morphology, patterns of cell surface markers, biochemical tests, immunohistochemical staining, and *ex vivo* colony formation assays.

Hematopoietic cells, including hematopoietic stem cells (HSC), are known to be adversely affected by many cancer therapies. While it is understood that traditional chemotherapeutic cancer treatments generally exert their adverse effect on hematopoietic cells of the bone marrow due to the rapidly dividing nature of the hematopoietic cells, alternative cancer therapeutics and non-cancer therapeutics, such as small molecule inhibitors, can also cause adverse effects on the hematopoietic system - albeit through different and often unknown mechanisms. Because the newly discovered bone marrow components of the present invention are identified herein as a source for new bone marrow formation and interact with and/or maintain populations of hematopoietic cells, hematopoietic stem cells, mesenchymal stem cells/progenitor cells, osteoclasts, and osteoblasts, methods of detecting compounds that adversely affect the bone marrow components are useful for the detecting whether a compound causes adverse effects on bone marrow homeostasis or hematopoiesis.

Adverse effects, for example on bone marrow homeostasis or hematopoiesis, as used herein, relates to unwanted or undesired effects on the bone marrow. Examples include reduction in or damage to the cells and bone marrow components of the bone marrow, affecting its ability to produce new blood cells. Because numerous hematopoietic cells are produced in the bone marrow, such adverse effects can lead to low red blood cell levels (anemia), which often results in varying levels of fatigue and weakness; low white cell levels (leukopenia, neutropenia, or granulocytopenia), which often leads to a reduced ability to fight infections as well as the inability to successfully repress any chronic infections (i.e., herpes, shingles, Epstein-Barr virus, HPV, hepatitis, HIV, etc.); and low levels of platelets (thrombocytopenia), which affect the blood's ability to clot and often leads to easy and/or excessive bruising, hemorrhaging, and internal bleeding.

As used herein "inhibit" and "inhibition" refer to, for example, stasis of symptoms, prolongation of survival, partial or full amelioration of symptoms, and partial or full eradication of a condition, disease or disorder. Inhibition of a bone marrow component as used herein relates to the stopping or reversing the growth or development of the bone marrow component. It further includes the reduction in the size of a bone marrow component or in the numbers of bone marrow components. It also includes the prevention of new growth or development of a new bone marrow component. It can also refer to a stasis of the bone marrow component function such as, for example, the recruitment or maintenance of hematopoietic cells within the bone marrow component. As used herein, reduction in growth, size, dilation and/or numbers includes, for example, a fold reduction such as about 2-fold, about 3-fold, about 4-fold, about 5-fold, about 10-fold, about 20-fold, about 50-fold, or any fold reduction in between. Similarly, reduction in growth, size and/or numbers can include a percent reduction of about 1%, 2%, 3%, 4%, 5%, 10%, 20%, 30%, 40%, 50%, 60%, 70%, 80%, 90%, 95% or any percentage reduction in between.

As further used herein, inhibiting cancer includes stasis, partial or total elimination of a cancerous growth or tumor. Partial elimination includes, for example, a fold reduction in growth or size and/or volume such as about 2-fold, about 3-fold, about 4-fold, about 5-fold, about 10-fold, about 20-fold, about 50-fold, or any fold reduction in between. Similarly, partial elimination can include a percent reduction in growth or size and/or volume of about 1%, 2%, 3%, 4%, 5%, 10%, 20%, 30%, 40%, 50%, 60%, 70%, 80%, 90%, 95% or any percentage reduction in between.

In a preferred embodiment of the inventions of a method for identifying compounds that inhibit cancer, a method for identifying whether a compound causes adverse side effects on bone marrow homeostasis or hematopoiesis, a method for identifying compounds that modulate the bone marrow component, and a method for identifying a compound that modulates angiogenesis, the subject has a non-solid cancer. And in a more preferred embodiment, the non-solid cancer is leukemia.

Because the bone marrow component of the invention is a precursor to the formation of bone marrow lacuna and thus trabecular bone, the bone marrow component can be useful as an indicator for identifying compounds which promote growth, expansion, or homeostasis of trabecular bone. Thus, an embodiment of the present invention also relates to a method for identifying a compound that promotes trabecular bone formation by detecting a bone marrow component within a bone marrow sample provided from a subject that has been previously administered the compound, wherein an increase in the number of the bone marrow components indicates that the compound promotes trabecular bone formation. In a preferred embodiment, the subject has osteoporosis.

As described herein, modulation of the bone marrow component affects the development, differentiation and maintenance of osteoprogenitor cells and subsequent trabecular bone formation. Trabecular bone, as used herein, relates to one of two types of osseous tissue that form bones. Compared to cortical bone, which is the other type of osseous tissue that forms the outer surface of bones, it has a higher surface area but is less dense, softer, and less stiff. Generally, trabecular bone comprises the established bone marrow found centrally within bones and extending outwards towards the bone ends and growth plates. It is also proximal from the bone marrow component of the present invention. Trabecular bone is also known to be the area/type of bone most commonly and immediately affected by osteoporosis. Thus, the discovery of the bone marrow component and its relationship to trabecular bone formation provides a novel method for identifying compounds which promote trabecular bone formation, thus also aiding in the treatment of bone diseases such as osteoporosis.

As used herein, promotion of trabecular bone formation relates to the creation of new or additional trabecular bone. It also relates to the slowing of, prevention of, or reversal of trabecular bone resorption, bone loss, bone demineralization, and/or disruption of bone micro-architecture. It further relates to the maintenance or preservation of trabecular bone homeostasis.

Various techniques are commonly known and used in the art for the detection of changes in bones. For example, nuclear medicine bone scans are useful and include magnetic resonance imaging (MRI), X-ray computed tomography (CT) and nuclear medicine bone scans (scintigraphy). Other techniques include dual-emission X-ray absorptiometry (DEXA or DXA), and Computed Quantitative Computer Tomography (QCT). Additional biologic assays for the detection of collagen breakdown products, and other biological factors associated with bone formation, resorption, and loss are also known in the art. Thus, numerous methods for detection of changes in bone and promotion of trabecular bone formation are useful with the methods of the present invention and are contemplated herein.

In a preferred embodiment of any of the method claims of the invention, detecting a bone marrow component or detecting a change in bone marrow component morphology comprises assaying bone marrow component development, assaying bone marrow component homeostasis, or assaying hematopoiesis. In a more preferred embodiment, assaying bone marrow component development, bone marrow component homeostasis, or hematopoiesis comprises at least one of detecting the frequency of bone marrow components, detecting the number of bone marrow components, and detecting the frequency or number of capillaries having at least part of an inner endothelial cell layer detached from an outer mesenchymal cell layer. Detecting bone marrow component morphology or changes in bone marrow component morphology can also include detecting hematopoietic stem cells (HSC) or long-term hematopoietic stem cells (LT-HSC) associated with the bone marrow component, detecting capillary endothelial cell growth, or detecting dilation of capillaries. Exemplary techniques for detection of a bone marrow component are described herein and shown by the examples and figures. Such techniques include, but are not limited to, electron microscopy, immunohistochemistry, and scanning confocal microscopy. Immunohistochemistry refers to the process of detecting antigens (e.g., proteins) in cells of a tissue section by exploiting the principle of antibodies binding specifically to antigens in biological tissues (tissues, cell cultures, etc.). Specific molecular markers are characteristic of particular cell types, proteins on or within cells, or cellular events such as proliferation or cell death (apoptosis). Immunohistochemistry is also widely used in basic research and clinical diagnosis to understand and identify the distribution and localization of biomarkers and differentially expressed proteins in different parts of a biological tissue. Visualizing an antibody-antigen interaction can be accomplished in a number of ways including, by way of example, where an antibody is conjugated to an enzyme, such as peroxidase, that can catalyze a color-producing reaction or where the antibody is tagged to a fluorophore or other detectable marker (metal particle, radio-label, etc.). Detection of the immunohistochemical stain is usually accomplished via standard detection for the given marker. Examples of detection methods include colorimetric or radiographic readers/detectors, colorimetric or radiographic assays, plate readers, fluorescence detectors (cameras, flow cytometers, etc.), and various microscopy techniques such as light microscopy, electron microscopy, confocal microscopy, or scanning confocal microscopy. The use of these, and other, microscopy techniques are well-established within the art and contemplated herein.

Additionally, stains and dyes can be used to highlight structures in biological tissues for viewing, often with the aid of different microscopes and other detection devices. Stains can be used to define and examine bulk tissues (highlighting, for example, bones, muscle fibers or connective tissue), cell populations (classifying different blood cells), or organelles within individual cells. It can further involve adding a class-specific (DNA, proteins, lipids, carbohydrates) dye to a substrate to qualify or quantify the presence of a specific compound. Numerous cell and tissue staining techniques are known within the art and are useful with the inventions described herein.

Detecting a bone marrow component, also relates to, but is not limited to, detecting the presence of a bone marrow component, the numbers of bone marrow components, the frequency of bone marrow components, the morphology of a bone marrow component, the development of a bone marrow component, the presence of mesenchymal cell types, the numbers and/or frequency of mesenchymal cell types, detecting capillary endothelial cells, detecting capillary dilation, detecting the frequency or number of capillaries having less than part of an endothelial cell layer detached from the mesenchymal cell layer, and detecting changes in the cellular and structural composition of the bone marrow component. Thus, detecting the morphology of a bone marrow component or detecting the development of a bone marrow component can also include detecting the presence or absence of the physical characteristics of the bone marrow component as described herein. Identification of the cells and structures which comprise the bone marrow component can be easily determined via the description of the bone marrow component herein and detection techniques known to one of skill in the art.

Detecting changes in the bone marrow component in certain embodiments, including changes in any of the previously mentioned embodiments of the methods or embodiments of the components, relates to detecting differences in the bone marrow component from the subject that received a compound as compared to the bone marrow component in a subject that did not receive the compound. Detecting changes also relates to changes in a bone marrow component as compared to established controls for a bone marrow component in a population comprising representative subjects. Detecting changes can further include detecting changes in a bone marrow component as compared to a bone marrow component from the same subject that were detected at an earlier or later time point, and/or that were detected prior to, and after, the administration of a compound to the subject.

In a preferred embodiment of any of the methods of the invention, assaying bone marrow component development, bone marrow component homeostasis, or hematopoiesis comprises at least one of detecting the frequency of Nestin+ mesenchymal cells, detecting the number of Nestin+ mesenchymal cells, detecting the frequency of F-actin protrusion-containing mesenchymal cells or osteoblastic cells, and detecting the number of F-actin protrusion+ mesenchymal cells or osteoblastic cells.

Nestin, as used herein, relates to a type VI intermediate filament protein. It is also referred to as NES or FLJ21841 and is known to form homodimers and homotetramers. Nestin is expressed by many types of cells during development, and the role of Nestin in dynamic cells, particularly structural organization of the cell, is regulated by phosphorylation, especially its integration into heterogeneous intermediate filaments such as vimentin or α -internexin. Mesenchymal cells such as mesenchymal stem cells or mesenchymal progenitor cells are known to express Nestin, and detection of Nestin can be achieved via techniques known in the art, including but not limited to, immunohistochemistry, microscopy, cytometry, or nucleic acid analyses (hybridizations, PCR, etc.).

F-actin, as used herein, relates to the polymeric form of G-actin and is part of a microfilament. Microfilaments are cytoskeletal structures and are one of the three major components of the cytoskeleton. G-actin (the monomer form) is transformed to F-actin (the polymer form) by ATP, where G-actin subunits assemble into long filamentous polymers called F-actin. Mesenchymal cells, such as mesenchymal stem cells/progenitor cells, osteo-progenitors, osteoblasts, osteoclasts, etc. are known to contain the cytoskeletal feature of F-actin protrusions. The presence of F-actin in cells can be easily detected/determined via techniques known in the art, including but not limited to, immunohistochemistry, microscopy, cytometry, or nucleic acid analyses (hybridizations, PCR, etc.).

In yet another preferred embodiment of any of the methods of the invention, assaying bone marrow component development, bone marrow component homeostasis, or hematopoiesis comprises at least one of detecting the frequency of hematopoietic stem cells (HSC) associated with a bone marrow component, detecting the number of HSC associated with a bone marrow component, detecting the frequency of long-term hematopoietic stem cells (LT-HSC) associated with a bone marrow component, and detecting the number of LT-HSC associated with a bone marrow component,. Assaying bone marrow component development, bone marrow component homeostasis, or hematopoiesis can also include detecting capillary cell growth, changes in capillary cell growth, and/or the dilation of capillaries. Assaying bone marrow component development, bone marrow component homeostasis, or hematopoiesis can still further include detecting the blood profile of a subject using established assays which identify the components, factors, and cell types present in a blood sample. Various assays are known within the art and routinely utilized to monitor hematopoietic status of subjects and are contemplated herein.

Detecting the frequency and/or number of Nestin+ mesenchymal cells, detecting the frequency and/or number of F-actin+ mesenchymal cells or osteoblastic cells can be achieved via numerous detection techniques known in the art. Similarly, detecting the frequency and/or number of hematopoietic stem cells (HSC) and detecting the frequency and/or number of long-term hematopoietic stem cells (LT-HSC) associated with bone marrow components can also be achieved by various detection techniques known in the art. As described herein and shown by the examples and figures, examples of such detection techniques include, but are not limited to, cytometric analysis (cell staining, flow cytometry, etc.), immunostaining, histochemistry, immunohistochemistry, and various types of microscopy (e.g., light, electron, etc.).

Homeostasis, as used herein, relates to the regulation of the equilibrium between different cell types of a bone marrow component. Multiple dynamic equilibrium adjustments and regulation mechanisms are involved in the homeostasis of a bone marrow component. It also relates to the physical development and maintenance of the bone marrow component, including any associated cells within and around the bone marrow component. Further included is the dynamic modulation of the bone marrow component, thus modulating the bone marrow structure. In accordance with the present invention, homeostasis and/or dynamic modulation of the bone marrow component relates to angiogenic growth, loss, and re-growth of sinusoidal vessels, which are covered by mesenchymal cells serving as osteoprogenitors, and the local separation of these two cell layers to generate a bone marrow component (i.e., hemospheres) that can accommodate hematopoietic cells of multiple types. Bone marrow component expansion and fusion processes create increasingly larger structures, which ultimately merge into a continuous bone marrow cavity and thus eventually form established bone marrow (i.e., continuous lacunal bone marrow spaces). Homeostasis also relates to the presence and interaction of the cells and structures which comprise the bone marrow component. Examples include the presence or absence of hematopoietic stem cells, mesenchymal cells, osteoblasts, osteoclasts, and endothelial cells

Hematopoietic stem cells (HSC's) can be identified by the presence of various antigenic markers on their surfaces, including but not limited to, CD34, CD59, Thy1/CD90, CD38, C-kit/CD117, CD133, CD105, CD48, and CD150. The hematopoietic stem cells are often negative for the markers that are used for detection of lineage commitment, and can thus also called Lin-. An additional system for stem cell identification known in the art uses a signature based on the SLAM family of cell surface molecules established by Morrison and colleagues (Kiel et al. 2005). The SLAM (signaling lymphocyte activation molecule) family is a group of molecules whose genes are located mostly in a single locus on chromosome, all belonging to a subset of immunoglobulin gene superfamily. This family includes, for example, CD48, CD150, CD244, CD34, CD16/32, SCA-1, c-kit, and Lin markers. "Lin" is known in the art to represent whether a cell expresses any number of markers that identify it as a member of a lineage-committed cell-type.

Long-term hematopoietic stem cells (LT-HSCs), in accordance with the present invention, relate to hematopoietic stem cells which have the ability of life-long self-renewal in addition to possessing the ability to differentiate into any blood cell type (i.e., multipotency). Identification and/or characterization of these cell types are also known in the art. An example of a characterization scheme is again the SLAM code, where LT-HSCs can be identified via the presence or absence of several markers including, but not limited to: CD34, SCA-1 , Thy1.1, C-kit, lin, CD135, SLAMF1/CD150, and SLAMF2/CD48.

Different SLAM codes are assigned to different categories of cell-types. SLAM codes can include, but are not limited to:
Hematopoietic stem cells (HSC): CD150+,CD48-,CD244-
Multipotent progenitor cells (MPPs): CD150-,CD48-,CD244+
Lineage-restricted progenitor cells (LRPs): CD150-,CD48+,CD244+
Granulocyte-macrophage progenitor (GMP): lin-,SCA-1-,c-kit+,CD34+,CD16/32hi Megakaryocyte-erythroid progenitor (MEP): lin-,SCA-1-,c-kit+,CD34-,CD16/32low
All the aforementioned markers for stem cells are useful for identifying the cells in the present invention. Any number of these markers can be used in a variety of combinations for the identification of a given stem cell type. Various combinations of markers for each stem cell type, in addition to those expressly described above, are known in the art and contemplated herein. Additional identification systems, surface antigens and other cellular or physical markers for the identification of HSC's are known in the art and are also useful with the present invention.

In another embodiment, the present invention also relates to the use of at least one ligand selected from Jagged1, DLL4, or VEGF is for modulating the bone marrow component of the present invention in a subject. DII4 activates Notch receptors in the adjacent (stalk) endothelial cells and can suppress VEGF receptor expression in these cells. A second Notch ligand, Jagged1, is highly expressed in stalk cells, antagonizes DII4-Notch signaling and thereby promotes angiogenic growth (Benedito et al., 2009). As described herein, genetic ablation of the gene encoding VEGFR2 (Flk1) in the postnatal endothelium compromises metaphyseal architecture and results in loss of trabecular bone, absence of a zone of SEC growth near the growth plate and the dilation of sinusoidal capillaries. Additionally, hematopoietic cells can be found in direct proximity of the growth plate and defined bone marrow components are absent. Similarly, inactivation of the Jag1 gene compromises femoral angiogenesis, sinusoidal endothelial cell (SEC) growth, maintenance of mesenchynmal progenitor cells, maintenance of LT-HSC, and trabecular bone formation, thereby interfering with bone marrow component formation, development and homeostasis. As also described herein, over-expression of VEGF-A leads to excessive sinusoidal vessel growth and the expansion of the metaphyseal (angiogenic) zone between the growth plate and the bone marrow. Thus, ligands of the Notch receptor and/or VEGF receptor can be useful for modulating the bone marrow component of the present invention. Furthermore, other compounds which are known to modulate Notch receptors or VEGF receptors can also be useful for modulating the bone marrow component described herein. Exemplary ligands and compounds include, but are not limited to, Jagged-1, Jagged-2, DLL1, DLL3, DLL4, VEGF-A, VEGF-B, VEGF-C, VEGF-D, and PIGF. Additional compounds include antibody therapeutics such as abevacizumab (Avastin), antibody derivatives such as ranibizumab (Lucentis), and small molecules that inhibit the tyrosine kinases stimulated by VEGF such as lapatinib (Tykerb), sunitinib (Sutent), SU5416 (Semaxanib), sorafenib (Nexavar), axitinib, and pazopanib.

Further homologs of known mammalian Notch ligands or VEGF ligands may be identified using standard techniques. By a "homolog" it is meant a gene or a gene product that exhibits sequence identity, either amino acid or nucleic acid sequence identity, to any one of the known Notch or VEGF ligands and has the same function of the known Notch or VEGF ligands. In accordance with the present invention, the term "% sequence identity" describes the number of matches ("hits") of identical nucleotides/amino acids of two or more aligned nucleic acid or amino acid sequences as compared to the number of nucleotides or amino acid residues making up the overall length of the nucleic acid or amino acid sequences (or the overall compared part thereof). In other terms, using an alignment, for two or more sequences or sub-sequences the percentage of amino acid residues or nucleotides that are the same (e.g., 80% or 85% identity) may be determined, when the (sub)sequences are compared and aligned for maximum correspondence over a window of comparison, or over a designated region as measured using a sequence comparison algorithm as known in the art, or when manually aligned and visually inspected. This definition also applies to the complement of a test sequence. Preferred nucleic acid molecules/polypeptides in accordance with the invention are those where the described identity exists over a region that is at least about 15 to 25 amino acids or nucleotides in length, more preferably, over a region that is at least about 50 to 100 amino acids or nucleotides in length. More preferred nucleic acid molecules/polypeptides in accordance with the present invention are those having the described sequence identity over the entire length of the nucleic acid molecule or polypeptide. Those having skill in the art will know how to determine percent sequence identity between/among sequences using, for example, algorithms such as those based on the NCBI BLAST algorithm (Stephen F. Altschul, Thomas L. Madden, Alejandro A. Schäffer, Jinghui Zhang, Zheng Zhang, Webb Miller, and David J. Lipman (1997), "Gapped BLAST and PSI-BLAST: a new generation of protein database search programs", Nucleic Acids Res. 25:3389-3402), CLUSTALW computer program (Thompson Nucl. Acids Res. 2 (1994), 4673-4680) or FASTA (Pearson and Lipman, Proc. Natl. Acad. Sci., 1988, 85; 2444), as known in the art.

The NCBI BLAST algorithm is preferably employed in accordance with this invention. The BLASTN program for nucleic acid sequences uses as default a word length (W) of 11, an expectation (E) of 10, M=5, N=4, and a comparison of both strands. For amino acid sequences, the BLASTP program uses as default a word length (W) of 3, and an expectation (E) of 10. The BLOSUM62 scoring matrix (Henikoff, Proc. Natl. Acad. Sci., 1989, 89:10915) uses alignments (B) of 50, expectation (E) of 10, M=5, N=4, and a comparison of both strands.

In accordance with the invention, transgenic non-human animals can be used with any of the methods of the invention. The transgenic non-human animals of the invention are prepared using standard methods known in the art for introducing exogenous nucleic acid into the genome of a non-human animal. In a preferred embodiment of any of the methods of the invention, the subject is a transgenic mouse. In a more preferred embodiment, the animal is a transgenic mouse which expresses markers for at least one cell-type found in or associated with a bone marrow component. In a most preferred embodiment, the transgenic mouse is the offspring of the combination of an endothelial-specific, tamoxifen-inducible Cdh5(PAC)-CreERT2 transgenic mouse and a mT/mG Cre reporter mouse. Such mice are known in the art and can be easily obtained and/or created via the use of standardized or commercial mouse strains and standard breeding techniques. An exemplary established method for preparing the transgenic non-human animal, in particular a transgenic mouse, is that of pronuclear microinjection (see e.g., Wagner, T. E. et al. (1981) Proc. Natl. Acad. Sci. USA 78:6376-6380). In general, the method involves introducing exogenous genetic material into the pronucleus of a mammalian zygote (e.g., mouse zygote) by microinjection to obtain a genetically transformed zygote and then transplanting the genetically transformed zygote into a pseudopregnant female animal. The embryo is then allowed to develop to term and the genome of the resultant offspring is analyzed for the presence of the transgenic material. Southern blot analysis, PCR or other such technique for analyzing genomic DNA is used to detect the presence of a unique nucleic acid fragment that would not be present in the non-transgenic animal but would be present in the transgenic animal. Selective breeding of transgenic offspring allows for homozygosity of the transgene to be achieved.

Other animal species that have immune, bone marrow and hematopoietic systems similar to, or are commonly used to represent human systems, may also be used. Examples of such species include but are not limited to rats, rabbits, chickens, goats, pigs, sheep and cows. Techniques for creating transgenic animals of each of these species have been described in the art. For example, preparation of transgenic rats is described in Tesson, L. et al. (2005) Transgenic Res. 14:531-546, including by techniques such as DNA microinjection, lentiviral vector mediated DNA transfer into early embryos and sperm-mediated transgenesis. Methods of transgenesis in rats are also described in Mullin, L. J. et al. (2002) Methods Mol. Biol. 180:255-270. Preparation of transgenic rabbits is described in, for example, Fan, J. et al. (1999) Pathol. Int. 49:583-594; Fan, J. and Watanabe, T. (2000) J. Atheroscler. Thromb. 7:26-32; Bosze, Z. et al. (2003) Transgenic Res. 12:541-553. Preparation of transgenic pigs is described in, for example, Zhou, C. Y. et al. (2002) Xenotransplantation 9:183-190; Vodicka, P. et al. (2005) Ann. N.Y. Acad. Sci. 1049:161-171. Alternative transgenesis techniques to pronuclear microinjection in pigs include adenovirus mediated introduction of DNA into pig sperm (see e.g., Farre, L. et al. (1999) Mol. Reprod. Dev. 53:149-158) and linker-based sperm-mediated gene transfer (Chang, K. et al. (2002) BMC Biotechnol. 2:5). Preparation of transgenic goats is described in, for example, Ebert, K. M. et al. (1991) Biotechnology (NY) 9:835-838; Baldassarre, H. et al. (2004) Reprod. Fertil. Dev. 16:465-470. Somatic cell nuclear transfer in goats is described in, for example, Behboodi, E. et al. (2004) Transgenic Res. 13:215-224. Preparation of transgenic sheep is described in, for example, Ward, K. A. and Brown, B. W. (1998) Reprod. Fertil. Dev. 10:659-665; Gou, K. M. et al. (2002) Shi Yan Sheng Wu Xue Bao 35:103-108 Preparation of transgenic cows is described in, for example, Donovan, D. M. et al. (2005) Transgenic Res. 14:563-567. Gene transfection of donor cells for nuclear transfer of bovine embryos is described in, for example, Lee S. L. et al. (2005) Mol. Reprod. Dev. 72:191-200. The state of the art in the preparation of transgenic domestic farm animals is also reviewed in Niemann, H. et al. (2005) Rev. Sci. Tech. 24:285-298. Preparation of transgenic chickens is described in, for example, Pain, B. et al. (1999) Cells Tissues Organs 165:212-219; Lillico, S. G. et al. (2005) Drug Discov. Today 10:191-196. Use of retroviral vectors in the preparation of transgenic chickens is described in, for example, Ishii, Y. et al. (2004) Dev. Dyn. 229:630-642.

The definitions as well as the preferred embodiments provided herein above with regard to other embodiments such as the bone marrow component of the invention apply mutatis mutandis also to the embodiments relating to the methods of identifying compounds that modulate bone marrow homeostasis, identifying compounds that modulate angiogenesis, identifying compounds the inhibit cancer, identifying compounds that promote trabecular bone formation, or identifying whether a compound causes adverse side effects on bone marrow homeostasis or hematopoiesis as outlined above.

Unless otherwise defined, all technical and scientific terms used herein have the same meaning as commonly understood by one of ordinary skill in the art to which this invention belongs. In case of conflict, the patent specification, including definitions, will prevail.

The figures show:

### Figure 1

### Angiogenic growth of the sinusoidal vasculature

(A-H) Three-dimensional tissue reconstitution of sinusoidal angiogenic growth in the distal femoral epiphysis (A-D) and sternum (E-H) of 1 month (A and E), 3 month (B and F), 5 month (C and G), and 7 month (D and H) old *Cdh5(PAC)-CreERT2 x ROSA26-mT*/*mG* mice. SECs (light grey), non-endothelial cells and cell nuclei (both dark grey) are labeled. Arrows indicate invading vessels, arrowheads vessel stalks and dotted lines the margin of the bone marrow (bm) cavity. Bone marrow in (A) is outside the field of view. Growth plate (gp) thickness in the femur decreases with age. Images in the right column (scale bar, 30µm) are higher magnifications of insets in panels on the left (scale bar, 100µm).
(I-K) Growth plate thickness (I) in relation to the length (distance between distal end and BM) of growing sinusoidal vessels (J) and the number of invading vessels in the growth plate (K) at the indicated ages. Error bars represent s.e.m.

### Figure 2

### Bone marrow is formed by bone marrow components (hemospheres)

(A-H) 3D reconstruction of bone marrow component formation in *Cdh5(PAC)-CreERT2* x *ROSA26-mT*/*mG* mice (scale bar, 30µm). SECs (light grey), non-endothelial cells and cell nuclei (both dark grey) are labeled; bone marrow (bm) and growth plate (gp) are indicated. (A) Individual planes of the Z-stack (right) and separated channels (center images) of a forming bone marrow component (inset in the 3D image shown on the left) showing the separation of the SEC and perivascular layers (elongated nuclei), which enclose a putative hematopoietic cell (round nucleus, arrow). Scale bars, 30µm.
(B-D) Larger bone marrow components (arrows) contain increasingly more CD45+ (lighter grey, marked by asterisks in C and D) hematopoietic cells. Non-endothelial cells (dark grey, arrowheads) in the periphery of bone marrow components are indicated.
(E) Branching and dilation of distal SEC capillary (top, arrows) and transversing vessel (arrowhead) passing by a cone-shaped SEC terminus (asterisks). Scale bar, 30µm.
(F) Bone marrow component in the sternum with GFP+ SEC terminus (light grey, lower arrow), a transversing vessel (light grey) connected to a distal, angiogenic SEC bulge (upper arrow), perivascular non-endothelial cells (dark grey, arrowheads) and hematopoietic cells (asterisks). Scale bars, 100µm and 30µm. Ribs are indicated. Right panel shows higher magnification of inset.
(G) Formation of bone marrow components in a secondary ossification center. Articular (ac) and growth plate (gp) cartilage, hematopoietic cells (asterisks), SECs (light grey, arrow), and perivascular non-endothelial cells (dark grey, arrowhead) are indicated. Scale bar, 100µm. Right panel shows higher magnification of inset.
(H) Bone marrow component in calvarium with a central SEC terminus (arrow), CD45+ (grey, asterisk) hematopoietic cells and peripheral non-endothelial cells (dark grey, arrowhead). Scale bars, 100µm and 30µm. Right panel shows higher magnification of inset.
(I) Two-photon microscopic image of a bone marrow component-like structure in human distal femur near the metaphysis. Von Willebrand Factor (VWF) expression marks SECs (ligh grey, arrows), collagen (2nd-harmonic signal) highlights trabecular bone (dark grey) and DAPI nuclei (intermediate grey). Arrowheads indicate perivascular non-endothelial cells and asterisks hematopoietic cells. Scale bar, 30µm.
(J-L) Electron micrograph of early (J; scale bar, 20µm) and larger (K and L; scale bar, 30µm) bone marrow components. Osteoblast (ob) and osteoclasts (oc) are indicated in (K). Right panel in (L) shows higher magnification of inset (scale bar, 20µm). Arrows indicate SECs, arrowheads perivascular non-endothelial cells and asterisks hematopoietic cells.

### Figure 3

### Localization and homing of putative HSCs

(A) Model illustrating principles of cavitating angiogenesis, bone marrow component ("hemosphere") initiation, expansion and fusion. SECs (innermost layer, light grey), mesenchymal cells (outer layer, darker grey), hematopoietic cells (round cells) and stem/progenitor cells (dark round cells) are shown.
(B) 3D reconstruction of 8-week old *Cdh5(PAC)-CreERT2* x *ROSA26-mT*/*mG* femur stained as indicated (treated with Tamoxifen and DMSO). Arrows mark the growing angiogenic front. Structures containing CD45+ (cyan) cells are smallest in proximity of the growth plate (gp) and become increasingly larger (arrowheads) close to the BM cavity (bm). Scale bar, 100µm.
(C) Immunofluorescence staining of fresh bone cryosections. Association of putative CD150+ (arrows) CD48- (dotted circles) HSC and CD150-/CD48+ progenitors (dark grey) with SECs (light grey) in *Cdh5(PAC)-CreERT2* x *ROSA26-mT*/*mG* femur. A megakaryocyte (Mk) is indicated. Scale bar, 30µm.
(D, E) Homing of transplanted EGFP-expressing (arrow) hematopoietic cells (stained with CD150 and CD48, as indicated) to hemospheres. ECs were stained with anti-VE-Cadherin antibody (light grey), nuclei with DAPI (dark grey). Scale bars, 30µm.
(F, G) Quantification of relative LT-HSC frequency in bone marrow components vs. established BM under homeostatic conditions (F) and after BM transplantation (G). Error bars represent s.e.m.

### Figure 4

### VEGF and Notch signaling control sinusoidal angiogenesis

(A-E) Immunofluorescence on thin sections of long bone with the indicated antibodies and reagents. F-actin was visualized with phalloidin (dark grey), SECs with anti-CD31 (light grey, arrows), and hematopoietic cells (asterisks) with anti-CD45 in (A, B and E). Nuclei, Hoechst staining. Right panels show higher magnifications of insets. Scale bars, 100µm (A, B, E), 30µm (C, D).
(A) EC-specific inactivation of VEGFR2 in 16-week old *Flk1*^{iΔEC}mutants disrupted angiogenesis, reduced trabecular bone (tb) to small islands, and led to dilation of vessels near the growth plate (gp).
(B) Expansion of the angiogenic growth zone containing SECs (arrows) near the metaphysis after VEGF-A overexpression (*VEGF-A*^{iGOF}).
(C) DII4 expression marked SECs near the growth plate (arrows) and elsewhere in the femur.
(D) Anti-Jagged1 immunofluorescence labeled angiogenic SECs (arrows) and a fraction of mesenchymal cells.
(E) Bone section from 18-week old *Jagged*1^{iΔEC} mutant showed defective angiogenesis, dilation of vessels and reduced trabecular bone (tb) compared to littermate control.

### Figure 5

### Regulation of mesenchymal progenitors by SECs

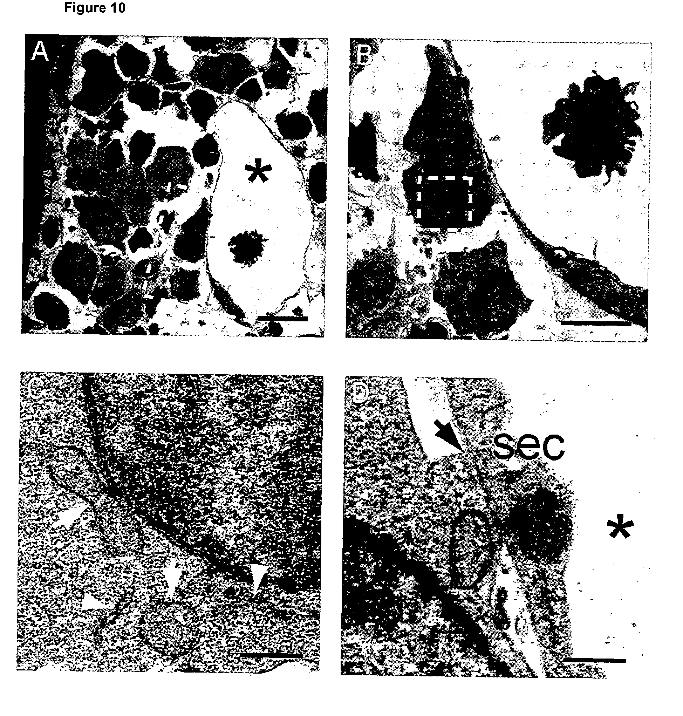

A) Schematic representation showing approximate regions of staining in the angiogenic front (as in Figure 10A), sinusoidal capillary stalk (B) and the endosteum (Figure 10H).
(B) Expression of NG2, CD146 and PDGFRβ (light grey signals, as indicated) in perivascular cells (arrows) surrounding the endothelium (arrowheads) in the stalk region. F-actin was visualized with phalloidin and nuclei by Hoechst staining (both dark grey). Scale bar, 30µm.
(C) Perivascular Nestin+ (light grey, arrows) cells are strongly reduced in *Jagged1*^{iΔEC} mutants (carrying a *mT*/*mG* Cre reporter allele). Trabecular bone (tb) is indicated.
(D) Overexpression of Jagged1 in ECs strongly amplifies the number of perivascular Nestin+ (light grey, arrows) compared to littermate controls.
(E) Angiogenesis in *VEGF-A*^{iGOF} femur leads to an expansion of perivascular Nestin+ (light grey, arrows) cells. Scale bars, 100µm.

### Figure 6

### Angiogenic signaling controls hemosphere morphology

(A-D) Morphological changes, visualized with the indicated markers, in femoral bone marrow components within the articular cartilage of *Flk1*^{iΔEC} (A), *DII4*^{iEC-GOF} (B), *Jagged1*^{iΔEC} (C), and *Jagged1*^{iEC-GOF} (D) mutants compared to littermate controls. Arrows indicate CD45+ (light grey) hematopoietic cells residing in between CD31+ SECs and the perivascular cells (dark grey). Scale bar, 30µm.
(E) Quantiation of CD45+ cells in mutant and littermate control bone marrow components. Error bars represent s.e.m.

### Figure 7

### Endothelial Jagged1 and VEGFR2 control HSC homeostasis

(A) Multi-parametric flow cytometry of whole bone marrow cells from *Jagged1*^{iΔEC} or littermate controls simultaneously stained with dye-coupled Lineage cocktail, c-Kit, Sca-1, Flk-2 and CD150 antibodies, as indicated. Black arrows denote the hierarchy of their parental subpopulations gated on certain antibody or their combinations. (B) Statistic analysis of LT-HSC frequency in *Jagged1*^{iΔEC} or littermate controls (n=3) following tamoxifen treatment for 1.5 months.
(C) Statistic analysis of LT-HSC frequency (left), absolute bone marrow cell (middle) and total LT-HSC numbers in each hindlimb of *Flk1*^{iΔEC} or littermate controls (control=4, mutant=3) at 5 months after tamoxifen administration
(D) Colony forming units (CFU) with multi-lineage (>3 lineages) potential (shown as colony forming cells (CFC) per 10⁶ BM cells) were reduced in *Flk1*^{iΔEC} mutants, whereas the number of total colonies and those derived from lineage-committed erythroid (CFU-E), granulocyte-macrophage (CFU-G/M) or granulocyte (CFU-G) progenitors were increased. No difference to control BM was observed for macrophage forming colonies (CFU-M). Error bars in (B-D) represent s.e.m.

### Figure 8

### Architecture of angiogenic sinusoidal vessels

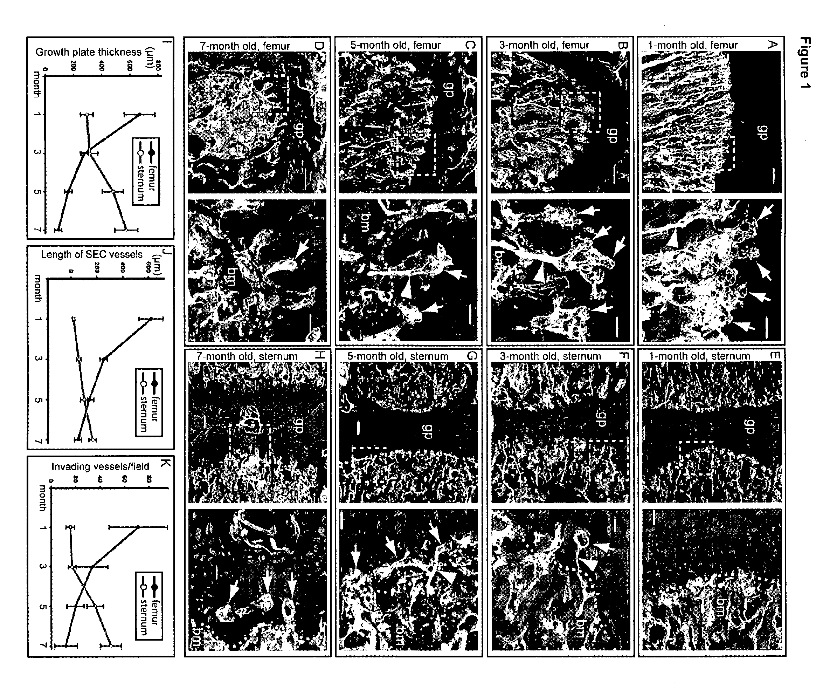

(A, B) Sprouting tip cells (arrows) in the mouse retina at postnatal day 6. ECs are labeled by isolectin B4 (light grey), red blood cells in the vessel lumen (asterisks) by unspecific fluorescence in the red channel (darker grey). Scale bar, 10µm.
(C, D) Individual planes of the confocal images shown in Figures 1C (C, femur) and 1G (D, sternum). SECs in *Cdh5(PAC)-CreERT2* x *ROSA26-mT*/*mG* mice (light grey, arrows), non-endothelial cells (darker grey, arrowheads) and nuclei were labeled. Chondrocytes (ch) are indicated. Distal (growing) sinusoidal vessels are fully lumenized (asterisks), lack tip cells and are associated with tomato+ (arrowhead) cells. Scale bars, 30µm.
(E-H) Electron micrographs of growing and fully lumenized (asterisks in E) sinusoidal vessels in direct proximity of growth plate chondrocytes (ch). Insets in (E) are shown in panels (F) and (G); inset in (G) is shown in (H). Osteoblast (ob) and osteoclasts (oc) are indicated. Scale bars, 25µm (E), 10µm (F, G), 5µm (H).

### Figure 9

### Organization of bone marrow components

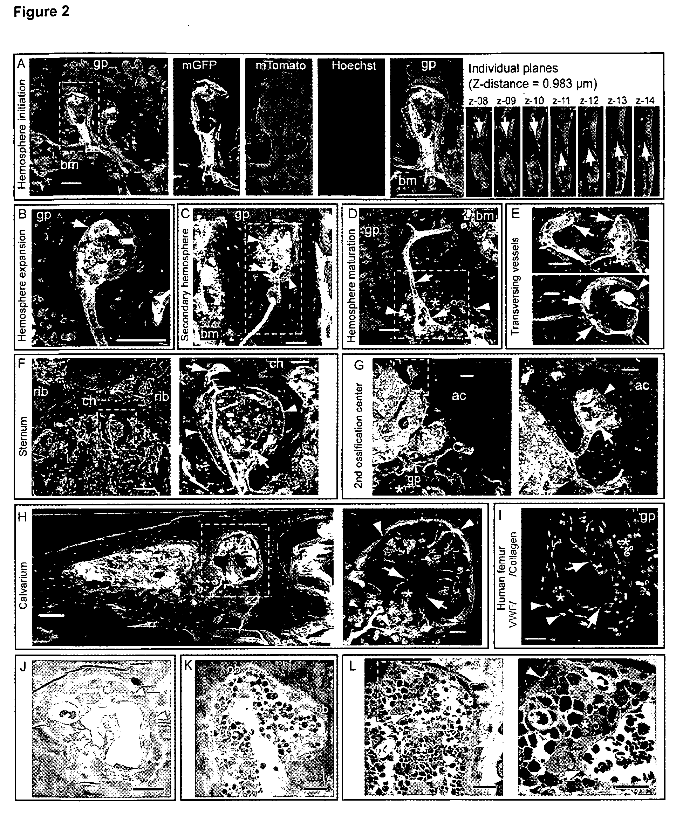

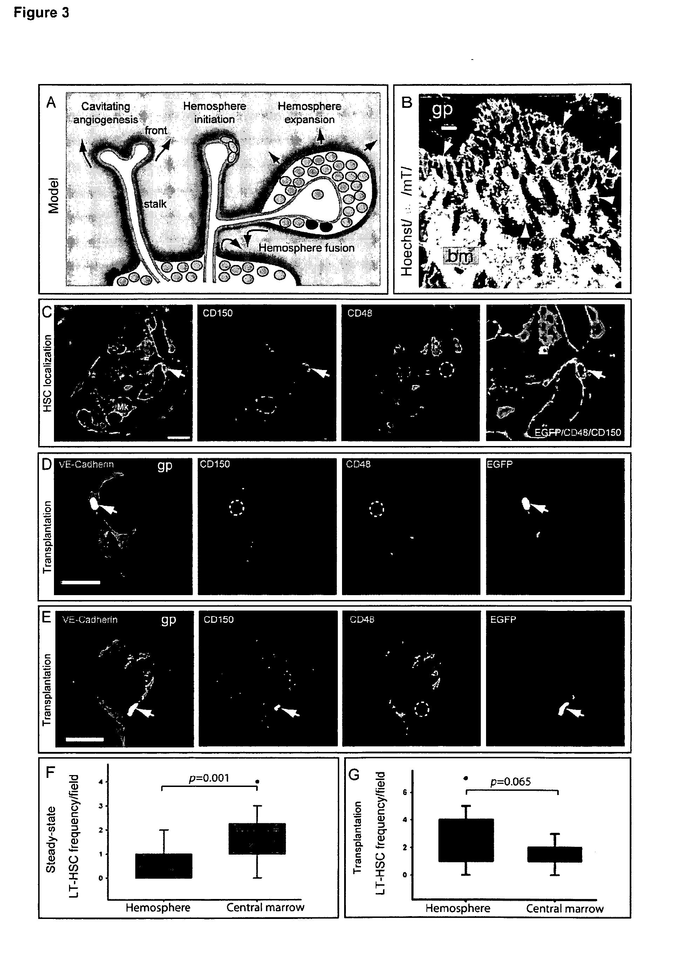

(A) Individual channels of inset in Figure 3C showing the expansion of CD45+ cells (cyan) in the growing bone marrow component. EGFP (light grey) labels endothelial cells, tomato (darker grey) non-ECs and Hoechst nuclei. Scale bar, 30µm.
(B) Selected planes from confocal Z-stack (image in Figure 2C) showing the separation of the SEC monolayer (light grey, arrows) and the perivascular cells (darker grey, arrowheads) as well as the presence of hematopoietic cells (asterisks).
(C) Separated channels of inset in Figure 2D showing the cone-shaped SEC terminus, the outer layer of non-ECs and CD45+ cells with roundish nuclei. Scale bar, 30µm.
(D) Selected planes from confocal Z-stack (image in Figure 2D) showing the separation of the SEC monolayer (arrows) and the perivascular cells (arrowheads) at the margin of the bone marrow component.
(E, F) Electron micrographs of sinusoidal vessels in the wild-type femur showing the presence of a lumen (asterisks) and physical interactions (arrows) between SECs and perivascular mesenchymal cells (mc). Osteoblasts (ob) and osteoclasts (oc) are indicated. Scale bars, 2µm.

### Figure 10

### Morphology of putative features of perivascular putative stem/progenitor cells in bone marrow components

(A-D) Electron micrograph of putative stem/progenitor cell largely devoid of rough endoplasmic reticulum (arrowheads) and with primitive mitochondria (arrows) (C). Note physical interaction (black arrow) with sinusoidal endothelium (sec) (D). (B) and (C, D) are higher magnifications of inset in (A) and (B), respectively. Asterisks marks lumen. Scale bar, 10µm (A), 5µm (B), 500nm (C, D).

### Figure 11

### Notch and VEGF signaling in the femur

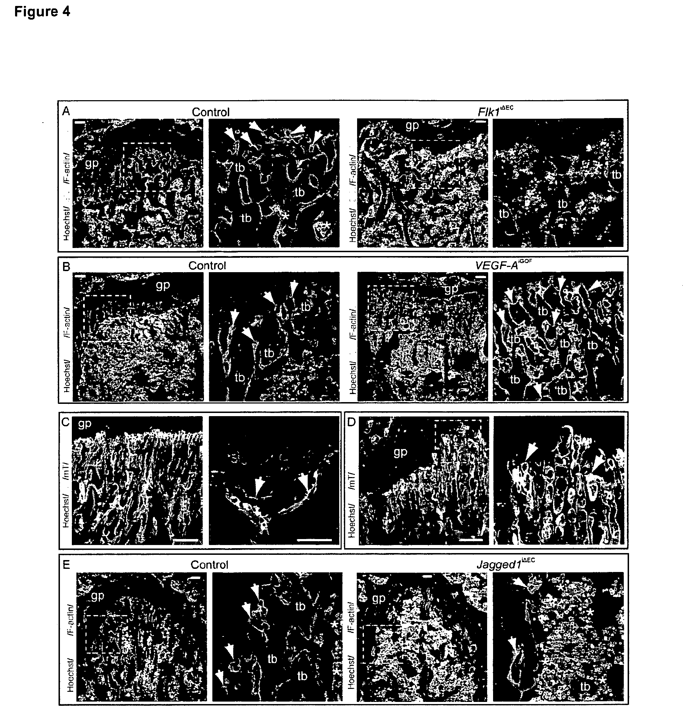

(A-C) Quantification of growing blood vessels and size of the angiogenic zone (i.e., distance between growth plate and BM) in *Flk1*^{iEC} (A), *VEGF-A*^{iGOF} (B) and *Jagged1*^{iEC} (C) mutants, as indicated. Error bars represent s.e.m.
(D, E) Separated channels of Figure 4C (D) and Figure 4D (E), as indicated.

### Figure 12

### Endothelial-mesenchymal interactions

(A) Expression of NG2, CD146 and PDGFRβ in perivascular cells (arrowheads) surrounding SECs (arrows) near the growth plate (gp) of the *Cdh5(PAC)-CreERT2 x ROSA26-mT*/*mG* femur. Scale bar, 30µm.
(B) Expression of CXCL12 (magenta) in perivascular cells of the stalk (near SECs, arrow) and other reticular cells (arrowheads).
(C) Cross-sections through *Cdh5(PAC)-CreERT2 x ROSA26-mT*/*mG* bone marrow components and immunofluorescence for Runx2, Osterix, Osteopontin (OPN) and N-Cadherin (light grey). Arrows point at positive cells (i.e., osteoblasts) in the periphery of the bone marrow components. EGFP-expressing SEC, non-endothelial cells and nuclei are also labeled. Scale bars, 30µm.
(D) Staining of mesenchymal progenitors (CD146) and Runx2-expressing hypertrophic and osteoblastic cells in the *Cdh5(PAC)-CreERT2 x ROSA26-mT*/*mG* femur, as indicated. Dotted line in the bottom right panel marks the interface between the major CD146 and Runx2 expression domains.
(E-H) Morphological changes affecting perivascular cells (arrowheads) in *Flk1*^{iΔEC} (E), *Jagged1*^{iΔEC}
(F), *Dll4*^{iEC-GOF} (G), and *Jagged1*^{iEC-GOF} (H) mutants in comparison to corresponding littermate controls. Endothelial cells (CD31, light green, arrows), actin (phalloidin, dark grew) and nuclei were stained. Scale bars, 30µm.

### Figure 13

### HSC homeostasis in endothelial mutants

(A) Multi-parametric flow cytometry of whole bone marrow cells from *Flk1*^{iΔEC} or littermate controls simultaneously stained with Lineage cocktail, c-Kit, Sca-1, Flk-2 and CD150 antibodies. Black arrows denote the hierarchy of their parental subpopulations gated on certain antibody or their combinations.

The examples illustrate the invention:

### Example 1:

### Experimental Procedures

### Mutant mice and inducible genetic experiments

For the overexpression of DII4 in ECs, a single copy of the full-length murine DII4 cDNA coupled to a tetracycline-responsive minimal promoter (Gossen and Bujard, 1992) was introduced into embryonic stem (ES) cells by targeted integration into the X-linked HPRT locus similar to what we have previously reported for *tetO-Jag1* trangenics (Benedito et al., 2009). Blastocyst injection of validated ES cell clones yielded *tetO-DII4* mice. For functional experiments, *tetO* lines were bred to VE-Cadherin-tTA transgenics (Sun et al., 2005). To avoid lethality triggered by DII4 or Jagged1 overexpression in the embryonic endothelium, females were given doxycycline (1mg/ml) in the drinking water throughout pregnancy. The generation of *VEGF-A*^{iGOF} mice and doxycycline-mediated induction of VEGF in the adult bone has been described previously (Maes et al., 2010).

For loss-of-function experiments, Flk1floxed/floxed (Haigh et al., 2003) or Jagged1floxed/floxed (Brooker et al., 2006) were combined with Pdgfb-iCreER (Claxton et al., 2008) or Cdh5(PAC)-CreERT2 (Wang et al., 2010) transgenic mice. The combination of the latter strain and Rosa26-mT/mG mice (Muzumdar et al., 2007) enabled the simultaneous labelling of the sinusoidal endothelium and the surrounding tissues. Experiments with inducible Cre transgenics in postnatal mice involved the intraperitoneal injection of tamoxifen solution (Sigma, T5648; 1mg/ml; generated by diluting a 10mg/ml tamoxifen stock solution in 1:4 ethanol:peanut oil) at a dose of 500µg/mouse/day for 5 days. Mouse offspring were routinely genotyped using standard PCR protocols. All animal experiments were performed in compliance with the institutional guidelines and were approved by local animal ethics committees.

### Bone immunohistochemistry and laser scanning confocal microscopy

Hind limb long bones or sternum were collected from mutant mice or their age-matched littermate controls at appropriate time points. Adherent muscles were trimmed, long bone epiphyses were split into halves, and sterna were divided into 3-4 pieces for maximal exposure of the marrow cavities. For 3D tissue reconstruction with the *Rosa26-mT*/*mG* Cre reporter strain, the collected bones were fixed, decalcified and permeabilized in a solution containing 2% paraformaldehyde (PFA), 10% EDTA and 0.25 Triton X-100 in PBS (pH 7.4) at 4°C for 3-7 days. Bone tissues were washed with PBS, embedded in 8% gelatin and stored at -80°C. 100-120µm thick sections were generated using low-profile blades and a Leica CM3050 cryostat.

For general morphological analyses of *Flk1*^{iΔEC}, *Jagged1*^{iΔEC}*,* DII4^{iEC-GOF} and Jagged1^{iEC-GOF} mutants and controls, thoroughly fixed and decalcified bone sections (8-10µm) were labelled with phalloidin-Alexa Fluor488 or 546 (1:500 dilution, Invitrogen) and Hoechst dye (1:1000, Invitrogen), and sequentially probed with rat anti-mouse CD31 (1:100, BD Pharmingen, Clone 13.3) and biotinylated rat anti-mouse CD45 monoclonal antibodies (1:200, BD Pharmingen, Clone 30-F11).

Thin cryosections of bone were prepared and stained. Freshly isolated distal ends of femurs were sliced longitudinally into fragments of approximately 0.5 × 1.0 × 2.0 mm and fixed in 0.5% PFA in PBS (pH 7.4) for 30 minutes at room temperature. The bone fragments were embedded in gelatin and snap-frozen in -80°C freezer. Sections (8µm) were generated with a tungsten carbide blade on a Leica CM3050 cryostat, air-dried, permeabilized in 0.25% Triton X-100, washed with PBS and blocked in 5% goat and/or rabbit serum at room temperature for 15mins. Blocked sections were probed with the following primary antibodies (diluted in 5% serum/PBS) for 1 hr at room temperature: CD150 (1:200, R&D Systems, cat. no. MAB4330), CD48 (1:500, R&D Systems, MAB3644), Jagged1 (1:200, R&D Systems; AF1277), DII4 (1:100, R&D Systems, MAB1389), NG2 (1:100, Millipore; AB5320), CD146 (1:200, Epitomics, EPR3208), PDGFR-β (1:200, eBioscience, APB5), Runx2 (1:200, R&D Systems; MAB2006), Osterix (1:50, Santa Cruz, sc-22536-R) Osteopontin (OPN, 1:100, R&D Systems, AF808), N-Cadherin (1:200, R&D Systems, MAB1388), CXCL-12/SDF-1 (1:50; Cell Signaling, D32F9), and Nestin (1:500; Millipore; MAB353). Afterwards, sections were washed with PBS for three times and incubated with appropriate Alexa Fluor-coupled secondary antibodies (1:500, Molecular Probes) for 1hr at room temperature. Sections were thoroughly washed, air-dried and sealed with anti-fade mounting medium (Invitrogen). The staining of the retinal vasculature and tip cells has been described elsewhere (Benedito et al., 2009). All immunofluorescent images were captured on a Leica SP5 inverted laser scanning confocal microscope. Z-stacks of images were processed and 3D reconstructed with Imaris software (version 7.00, Bitplane).

Human femur biopsies were taken from a patient (female at the age of 13 yrs 8 months, Tanner pubertal stage B3) who was undergoing surgery because of medical indications. Informed consent was obtained from the patient and the parents, and approval was given by the Local Medical Ethics Committee at The Karolinska University Hospital. Paraffin sections were de-waxed and subjected to antibody staining according to standard protocols (Emons et al., 2009). Two-photon microscopy was performed on human femur samples using a customized 2-photon microscope (TriM Scope II, LaVision BioTec) with detection by an array of independent photo-multiplier tubes (PMT) detectors with single-point illumination using a 20x Olympus objective (NA=1.0).

### Electron microscopy

For electron microscopy, the femur bone of 6-week old mice was processed similar to a protocol published by Suzuki et al. (Suzuki et al., 2005) with some modifications. The bone was removed and directly transferred into 4% paraformaldehyde, 0.5% glutaraldehyde in 0.1M cacodylate buffer (pH 7.2), cut longitudinally in two halves and fixed initially for 2 hrs at room temperature and overnight at 4°C under agitation. The sample was decalcified in 5% EDTA in 0.1 M cacodylate buffer (pH 7.2) at 4°C for 3 days with one exchange of the solution after 1.5 days. The sample was post-fixed for 1.5hrs in 1% OsmO4, 1.5% potassium ferrocyanide. Dehydration and embedding in epon was done under slight vacuum (Grills and Ham, 1989). 70nm ultrathin sections of the sample were taken in the area of interest and collected on filmed single slot copper grids (Leica UC6 ultramicrotome, Vienna, Austria). Sections were counterstained with uranyl-acetate and lead, and were analyzed at 80 kV on a FEI-Tecnai 12 electron microscope (FEI, Eindhoven, Netherlands). Pictures were taken with imaging plates (Ditabis, Pforzheim, Germany).

### Multiple parameter flow cytometry

Flow cytometric analysis of LT-HSCs was essentially performed as previously described (Kiel et al., 2005). Briefly, whole bone marrow cells were flushed from the hindlimb with Hank's buffered salt solution supplemented with 2% heat-inactivated fetal bovine serum (Invitrogen, HBSS). To obtain a single cell suspension, cells were passed through 27.5 gauge needles twice and filtered through a nylon screen (60µm mesh, Millipore). Cells were counted by trypan blue and a total of 10 x10⁶ cells were subjected to surface staining. The cell pellets were first blocked with the BD Fc Block CD16/32 antibody (BD Pharmingen) on ice for 5mins before staining with the lineage-APC cocktail, c-Kit-PE-Cy7, Sca-1 PerCP-Cy5-5, Flk-2-PE and CD150-AlexaFluor 488 simultaneously on ice for 15mins. The stained cells were washed twice with HBSS, processed by a FACSCanto workstation and analyzed with FACSDiva software (BD Bioscience). Flow plots were manually compensated.

### Competitive repopulation and colony formation assays

Eight week old C57BI6 female mice received a lethal dose of γ-irradiation (9.5gy 137Cs), within 24 hrs 1 x 10⁶ GFP-expressing bone marrow cells were infused via the lateral tail vein either in combination with 1 x 10⁶ or 2.5 x 10⁶ bone marrow cells derived from mutant or littermate control animals of the indicated experimental groups. After 4 weeks, animals were humanely sacrificed and the relative abundance of GFP-positive vs. negative BM cells was analyzed by FACS.

For colony formation assays, single bone marrow cells were seeded at 3 x 10³, 1 x 10⁴ and 3 x 10⁴ cells/ml in 35mm dishes into semisolid medium (IMDM, GIBCO) containing 1% (w/v) methylcellulose (Shin-Etsu-Chemical), 10% heat-inactivated foetal bovine serum (PAA), 5% deionised and delipidated BSA (Sigma), 10⁻⁴M β-mercaptoethanol, 1.5% X63-Ag-653 cell-conditioned medium (as a source of IL-3), 15% 5637 cell-conditioned medium, 200µg/ml iron-saturated transferrin (Sigma), 10µg/ml cholesterol, 0.25µg/ml linoleic acid, 0.25µg/ml oleic acid (Sigma), 5U/ml rhuEpo (Janssen Cilag), 10µg/ml rh insulin (Sigma), 12.5ng/ml rmSCF (Cell Systems), 10ng/ml rh IL-6 (Cell Systems). After 10 days, colonies consisting of more than 50 cells were scored and classified. Correct designation was confirmed by pan-chromatic staining of cytospin preparations. Duplicates of 3 concentrations were scored for each group.

### Natural bone culture

Bone cultures are performed by excising the desired limbs or bone structures from subjects such as mice. The limbs are carefully cleaned from the surrounding tissue (skin and muscles) and the desired bones exposed. The bones are then further cleared from tissue remains and ligaments. The bones are grown in an known growth medium (for example, α-MEM supplemented with penicillin (100 units/ml), streptomycin (0.1 mg/ml), nystatin (12.5 units/ml), BSA (0.2%), O-glycerophosphate (1 mM) and freshly prepared ascorbic acid (50 µg/ml)) in a tissue culture dish, and medium replacement is performed every three days. Test compounds are administered to the cultures at pre-determined concentrations and intervals and biopsies are obtained throughout the culture period to examine the effects of the test compounds on the bone marrow structures within the bone cultures.

### Example 2

### Angiogenic growth of the sinusoidal vasculature

To determine the tissue architecture of bone marrow, endothelial-specific, tamoxifen-inducible Cdh5(PAC)-CreERT2 transgenics (Wang et al., 2010) were combined with mT/mG Cre reporter mice (Muzumdar et al., 2007). While the resulting offspring displayed general expression of membrane-targeted tomato protein (mT), administration of tamoxifen and activation of Cre recombinase led to the excision of the mT cassette allowing the expression of membrane-targeted enhanced green fluorescent protein (mG) in endothelial cells (Figure 1A-H). This method permitted the detailed analysis of the sinusoidal endothelium and the surrounding tissue, thus identifying the bone marrow component of the present invention.

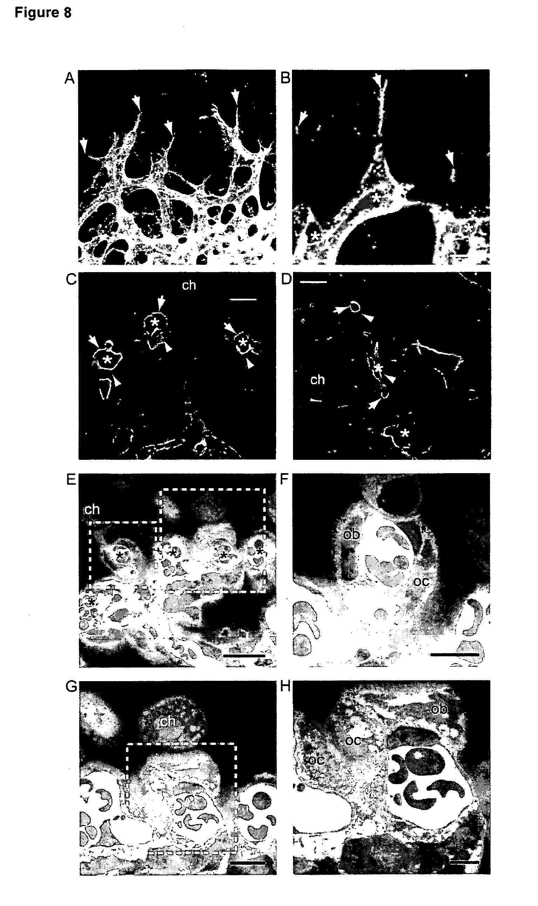

Analysis of the femur of adolescent (1 month old) animals showed high density and pronounced growth of the bone marrow components of the present invention near the growth plates, the sites of bone elongation (Figure 1A). However, in contrast to angiogenic growth in the retina, skin or other soft tissues, sinusoidal vessels lacked identifiable tip cells. Instead, the sinusoidal endothelium extended blunt and lumen-containing protrusions near the growth plate (Figure 8A-H). These distal vessels, representing the bone marrow components of the present invention, filled spaces created by chondrocyte apoptosis and osteoclast-mediated bone resorption in the growth plate and were connected to the adjacent bone marrow by long stalk-like capillaries (Figures 1A-C). Growth of the bone marrow components in the femur gradually decreased over time and only a few SEC protrusions were seen at the age of 7 months when the thickness of the growth plate had decreased significantly (Figure 1A-D and 1I-K). In contrast, only few sinusoidal tubules protruded into the sternal cartilage of adolescent mice despite the abundance of vessels in the adjacent ossified areas (Figure 1E). Invasion of the bone marrow components into the hypertophic chondrocyte layer was visible in 3 and 5-month old mice and was most pronounced in the sternum of the oldest mice analyzed (Figure 1E-H). Thus, the growth of the bone marrow components of the present invention in mice is inversely regulated in the femur and sternum between 1 and 7 months (Figure 1I-K). Furthermore, this temporal pattern correlates with known changes in the hematopoietic activity of the human skeleton where active marrow declines in the femur after adolescence and become restricted to other sites such as sternum, ribs, vertebrae, skull and pelvis (Kaushansy et al., 2005).

### Example 3

### Bone marrow is formed by bone marrow components ("hemospheres")

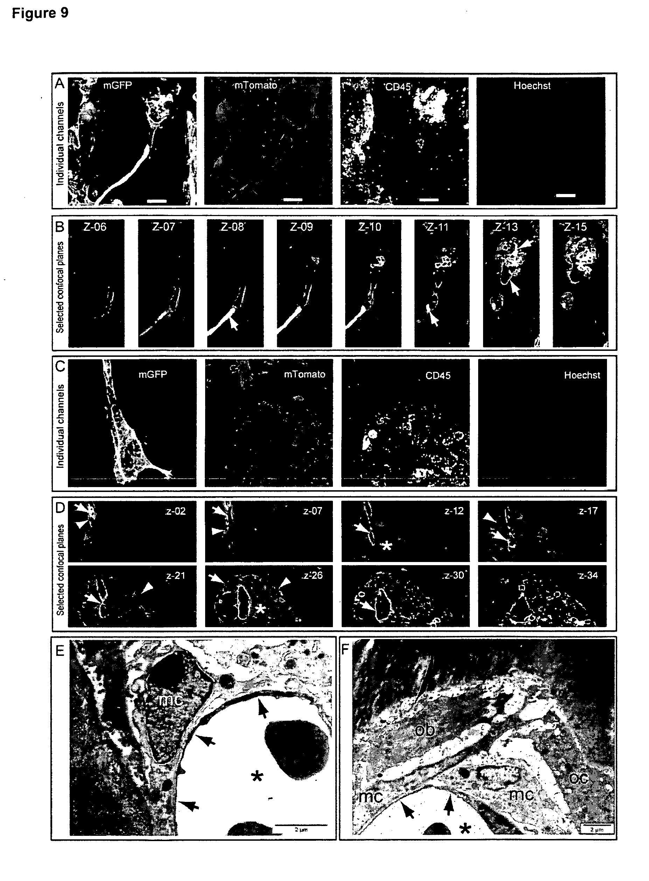

During our analysis of the vasculatures of long bones and sternum, we found that some vessels terminated in spheroid bulges adjacent to growth plate chondrocytes (Figure 2A). While the majority of growing sinusoidal vessels remained surrounded by perivascular, tomato-positive (non-endothelial) cells, this layer detached from the endothelium in some of the enlarged bulges and additional cells, which had round nuclei and belonged to the hematopoietic lineage, appeared in the resulting space (Figure 2A). In larger bulge structures, the area between SECs and perivascular cells was enlarged and, concomitantly, the number of the enclosed CD45+ hematopoietic cells increased (Figure 2B and C). These spherical structures represented the hematopoietic cell-containing sites closest to the growth plate and they represent bone marrow components of the present invention. Additional bone marrow component bulges were occasionally observed in the capillary providing the connection to the existing bone marrow (Figure 2C; Figure 9A and B), and these too are encompassed by the bone marrow components of the present invention. In structures exceeding an outer diameter of about 150µm, the central vessel terminated in a blind-ending, cone-shaped structure with a diameter of about 60-120µm (Figure 2D; Figure 9C and D). The endothelial cells of this terminal cone retained the thin, elongated morphology characteristic for SECs, but they lacked perivascular cell coverage (Figure 2D and 2J-L; Figure 9C and D).

Ultrastructural analysis also showed that the cone-shaped SEC terminus was tightly associated with surrounding hematopoietic cells (Figure 2J-L), while the endothelium of the stalk made physical contact with perivascular mesenchymal cells (Figure 9E and F). Further examination of the femur and sternum revealed that larger bone marrow components contained additional vessels (with a typical caliber of around 10µm). some of which were covered by tomato-positive (non-endothelial) cells, crossed the full length of the hematopoietic cell-filled spheroid and exited at the most distal surface (Figure 2E and F). Once these transiting vessels entered the cartilaginous tissue, they displayed the blind-ending, dilated and lumenized morphology of growing sinusoidal endothelium seen elsewhere (Figure 2E and F). Bone marrow components were also observed in the sternum (Figure 2F), in the epiphyseal cartilage of secondary ossification centers (Figure 2G), the skull (Figure 2H) as well as in human femur (Figure 2I). This shows that the bone marrow components reflect a general bone marrow process and are shared by different BM-containing skeletal elements and mammalian species.

While the smallest bone marrow components were found next to the femoral growth plate or the sternal cartilage, increasingly larger and interconnected bone marrow components were found in close proximity of the established bone marrow cavity suggesting that bone marrow component expansion and fusion processes ultimately lead to the generation of a continuous lacunal BM space (Figure 3A and B). Moreover, in the femur of 5 or 7 month-old mice (Figure 1C and D), the few blunt-ended vessels were located in direct proximity (in a distance of less than 50µm) of the bone marrow lacuna. The experiment demonstrates a sequential model of bone marrow component initiation, expansion, functional specialization and fusion, which leads to the generation of new bone marrow in the adult skeleton (Figure 3A).

### Example 4:

### Localization and homing of putative HSCs

Since CD45+ hematopoietic cells were highly abundant in bone marrow components (Figure 2B-D and H), we reasoned that hematopoietic stem and progenitor cells might be also present. To address this question, we stained thin tissue sections with antibodies against CD48/SLAMF2 and CD150/SLAMF1, which are members of the SLAM (signaling lymphocyte activation molecule) family of cell surface molecules. According to the SLAM code established by Morrison and colleagues (Kiel et al., 2005), we found that the infrequently occurring CD150+/CD48- cells, i.e., putative long-term hematopoietic stem cells (LT-HSCs), occur within bone marrow components (Figure 3F). These cells were physically associated with the outer surface of the cone-shaped SEC terminus (Figure 3C). Megakaryocytes were also CD150+/CD48- but were readily distinguishable from putative HSCs because of their large size and nuclei (Figure 3C). In addition, bone marrow components also contained a large number of CD150-/CD48+ lineage-restricted progenitor cells (Figure 3C). Ultrastructural examination of bone marrow components confirmed that cells displaying characteristic features of stem and progenitor cells, such as the low abundance of cellular organelles and the globular shape of mitochondria (Zeuschner et al., 2010), were found in physical contact with inner cell layer of endothelial cells (Figure 10A-D).

Following the transplantation of a 1:1 mixture of GFP-expressing and unlabeled bone marrow into irradiated recipients, GFP+ hematopoietic cells were found in the expected 50% ratio within the established bone marrow and larger (>150µm, outer diameter) bone marrow components. In contrast, small (<30µm, outer diameter) bone marrow components contained individual GFP+ cells in close association with SECs and perivascular cells (Figure 3D and E), which resembled newly forming bone marrow components at the initiation stage (Figure 2A). In a fraction of these cases, the bone marrow component-occupying cell was CD150+/CD48- (Figure 3E). The overall frequency of transplanted putative LT-HSCs within bone marrow components was higher than in the bone marrow cavity (Figure 3G). Thus, transplanted hematopoietic cells home to bone marrow components and occupy these structures, thus reinitiating bone marrow homeostasis.

### Example 5:

### VEGF and Notch signaling control sinusoidal angiogenesis

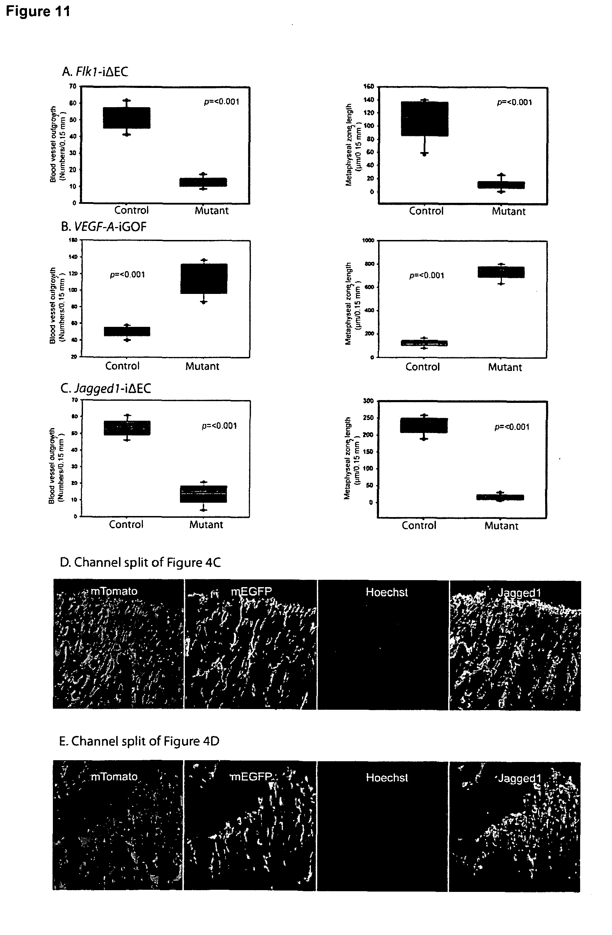

To test whether the growth of sinusoidal vessels and bone marrow component morphogenesis might be linked, we employed inducible gene targeting to interfere with angiogenesis in adolescent and adult mice. Genetic ablation of the gene encoding VEGFR2 (*Flk1*) in the postnatal endothelium with the help of the *Cdh5(PAC)-CreERT2* transgenic line strongly compromised metaphyseal architecture in the resulting *Flk1*^{iΔEC} mutants, which showed loss of trabecular bone, absence of a zone of SEC growth near the growth plate and the dilation of sinusoidal capillaries (Figure 4A, Figure 11A). These defects copied many phenotypic features previously reported for VEGF mutants (Maes et al., 2002) and of mice treated with SU5416 (Semaxanib), a highly potent and selective small molecular weight inhibitor of the VEGFR2 kinase (data not shown). Owing to an abnormal enlargement of the *Flk1*^{iΔEC} BM lacuna and the reduction of the surrounding bone, hematopoietic cells in mutants were found in direct proximity of the growth plate and defined bone marrow components were absent (Figure 4A, Figure 11A). Conversely, overexpression of VEGF-A in an inducible gain-of-function model (VEGF-A^{iGOF}) (Maes et al., 2010) for as little as four days led to excessive sinusoidal vessel growth and the expansion of the metaphyseal (angiogenic) zone between the growth plate and the bone marrow (Figure 4B, Figure 11B).

Previous work has shown that VEGF-regulated angiogenesis is strongly controlled by Notch signaling interactions in ECs (Hellstrom et al., 2007). DII4 is a marker of sprouting tip cells in the mouse retina and other tissues, activates Notch receptors in the adjacent (stalk) ECs and is thought to suppress VEGF receptor expression in these cells. A second Notch ligand, Jagged1, is highly expressed in stalk cells, antagonizes DII4-Notch signaling and thereby promotes angiogenic growth (Benedito et al., 2009). We found that Jagged1 and DII4 were also expressed in the femoral vasculature. However, DII4 protein was present in all vessels including the distal, angiogenic SECs and the long capillary stalks (Figure 4C, Figure 11D). In contrast, anti-Jagged1 immunofluorescence labeled the growing sinusoidal endothelium next to the growth plate as well as some associated non-endothelial cells (Figure 4D, Figure 11E). Cdh5(PAC)-CreERT2-mediated inactivation of the Jag1 gene compromised femoral angiogenesis similar to what we have previously reported for the retinal and dermal vasculature (Figure 4E). Like in *Flk1*^{iΔEC} mutant mice, SEC growth and trabecular bone formation were compromised, and hematopoietic cells reached up to the growth plate (Figure 4E).

### Example 6

### SECs control the phenotype of perivascular mesenchymal cells

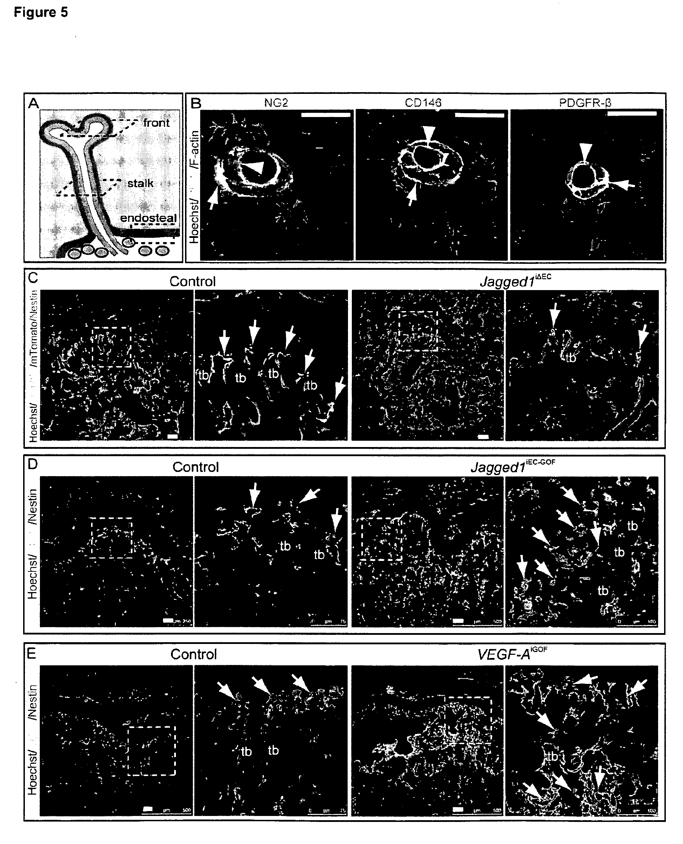

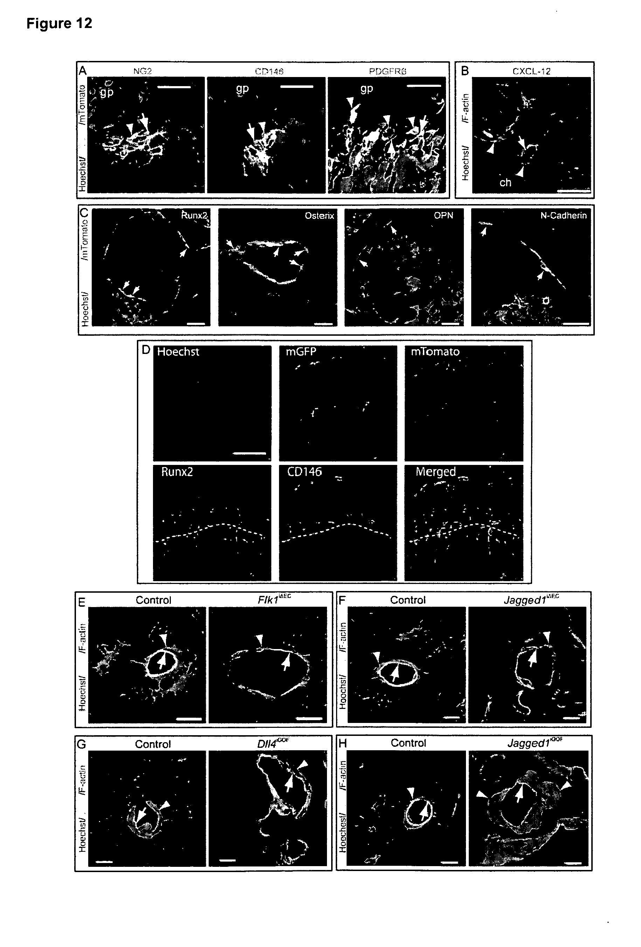

With the exception of the cone-shaped endothelium within hemospheres and the most distal end of growing sinusoidal vessels in direct proximity of the growth plate (Figure 1A, 1B and 2D), GFP-expressing SECs in *Cdh5(PAC)-CreERT2* x *mT*/*mG* reporter mice were tightly associated with surrounding non-endothelial (tomato-positive) cells (Figure 2C and 2D). Characterization of these perivascular cells revealed prominent expression of markers characteristic for mesenchymal cells and pericytes, the latter of which are thought to stabilize the blood vessel wall and thereby reduce vascular permeability (Armulik et al., 2005). Specifically, cells associated with SECs near the growth plate and those covering the adjacent capillary stalks (Figure 5A and B, Figure 12A) expressed the pericyte markers NG2 and platelet-derived growth factor receptor β (PDGFRβ) (Armulik et al., 2005). Moreover, we found strong perivascular expression of CD146 and Nestin (Figures 5B and 5C, Figure 12A), which are markers of mesenchymal stem cells in the bone marrow and elsewhere (Crisan et al., 2008; Mendez-Ferrer et al., 2010). Suggesting that these mesenchymal cells are osteoprogenitors that differentiate into osteoblasts and osteocytes (Erlebacher et al., 1995; Sacchetti et al., 2007), CD146 and the osteoblast differentiation factor Runx2 labeled complementary cell populations and showed only very limited overlap at the interface between the main expression domains (Figure 12C). The peripheral (tomato+, non-endothelial), bone marrow component-enclosing cells (the outer layer of the capillary of the present invention) also expressed osteoblast markers such as Runx2, osterix, N-cadherin and osteopontin (Figure 12B). Anti-CXCL12 immunofluorescence labeled the osteoblasts in the periphery of hemospheres as well as perivascular (progenitor) cells, which suggests that CAR cells might represent a rather heterogeneous cell population in femoral metaphysis of the mouse (Figure 12B).

The physical contact between perivascular, mesenchymal cells and SECs (Figure 9E and F) indicates the possibility of direct, cell-cell contact-mediated signaling interactions. Indeed, while the mesenchymal and osteoblastic cells surrounding control sinusoidal capillaries were rich in F-actin-containing protrusions, these cytoskeletal features were lost in endothelial-specific *Flk1*^{iΔEC} and *Jag1*^{iΔEC} mutant mice (Figure 12E and F). Likewise, transgenic overexpression of DII4 (DII4^{iGOF}) in the endothelium of adult mice increased the caliber of sinusoidal capillaries and led to the disappearance of F-actin protrusions in perivascular cells (Figure 12G). Conversely, Jagged1 overexpression (*Jag1*^{iGOF}), which affected sinusoidal angiogenesis only mildly (data not shown), caused a substantial accumulation of perivascular cells, which were present in multiple layers but lacked actin-rich protrusions (Figure 12H). These morphological changes were accompanied by altered expression of mesenchymal markers. The abundance of perivascular Nestin staining in the proximity of the femoral growth plate was strongly reduced in EC-specific *Jag1*^{iΔEC} mutants (Figure 5C). Conversely, the multiple ectopic layers of mesenchymal cells surrounding Jagged1-overexpressing (*Jag1*^{iGOF}) sinusoidal capillaries showed pronounced Nestin expression (Figure 5D). In a model of inducible VEGF-A overexpression in the bone (Maes et al., 2010), dramatic growth of the sinusoidal endothelium was also accompanied by the strong expansion of associated Nestin+ mesenchymal cells (Figure 5E). These results indicate that signals from the endothelium control the behavior of the perivascular mesenchyme. Consistent with previous work, which has established that Notch signaling promotes osteoprogenitor proliferation and suppresses osteoblast differentiation (Engin et al., 2008; Hilton et al., 2008), these results show that the presentation of Notch ligands by endothelial cells plays a key role in the maintenance of mesenchymal stem and progenitor cells in the bone marrow.

### Example 7:

### Angiogenic signaling controls bone marrow component morphology

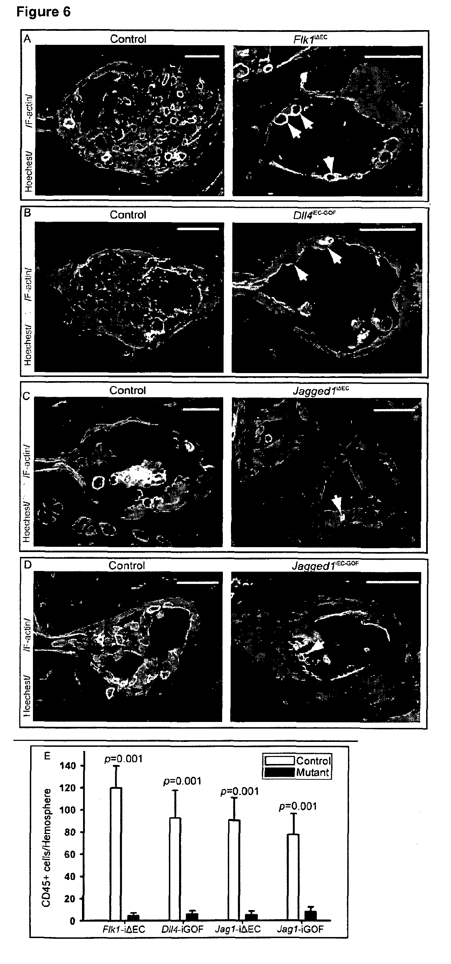

The data above show that VEGF and Notch signaling control the growth of the sinusoidal endothelium. Moreover, the differentiation of mesenchymal cells is influenced by local interactions with the endothelium, which is consistent with previous observations in VEGF mutant mice (Maes et al., 2002). As SECs, mesenchymal cells and osteoblasts are also major cellular components of hemospheres, we investigated how the manipulation of angiogenic pathways in the endothelium would affect the architecture of these structures. This was only possible in the mutant secondary ossification centers, because morphologically defined bone marrow components were absent in the *Flk1*^{iΔEC} and *Jag1*^{iΔEC} metaphysis (Figure 4A and E). In secondary ossification centers, the genetic inactivation of VEGFR2 in ECs led to the emergence of 'empty' bone marrow components, in which CD45+ hematopoietic cells were present at low abundance (Figure 6A and 6E). Likewise, EC-specific over-expression of DII4, which has been shown to inhibit angiogenesis in various other vascular beds (Li et al., 2007; Trindade et al., 2008; Williams et al., 2006), induced an empty bone marrow component phenotype (Figure 6B and 6E). Consistent with the observation that endothelial Jagged1 controls the differentiation of perivascular cells, EC-specific loss-of-function mutants at 8 weeks after Jag1 inactivation showed a full disruption of normal bone marrow component architecture and the disappearance of hematopoietic cells (Figure 6C and 6E). Conversely, the bone marrow components of *Jag1*^{iGOF} mice were thick-walled because of a strong accumulation of perivascular cells (Figure 6D) similar to what we had observed around mutant capillary stalks (Figure 12H). The sum of these results shows that sinusoidal endothelial cells provide important instructive signals for perivascular osteoprogenitor and osteoblast populations and that these interactions modulate bone marrow component formation.

### Example 8:

### Angiogenic pathways control hematopoietic stem cell homeostasis

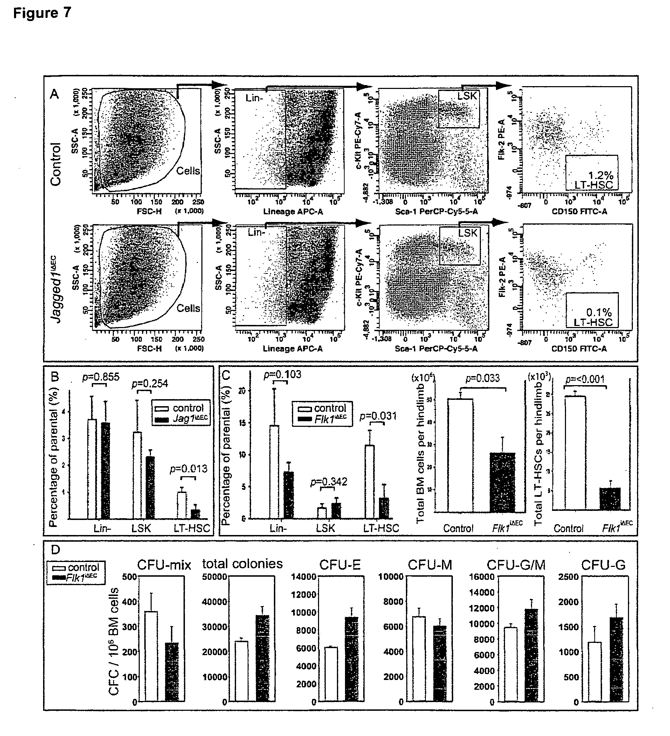

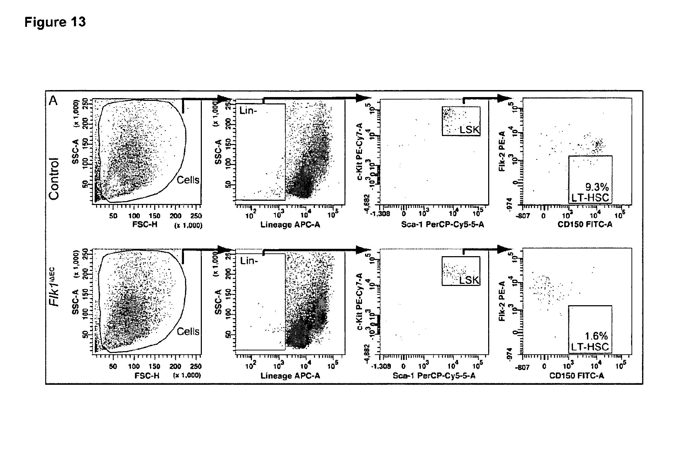

Given the dramatic morphological alterations of bone marrow components ("hemospheres") in the femur of *Flk1*^{iΔEC} and *Jag1*^{iΔEC} mutant mice and the absence of hematopoietic cells in these structures, we analyzed the LT-HSC population in these genetic models. Flow cytometry analysis of hematopoietic cells isolated from the hind limbs of control mice showed the expected small fraction of lineage (lin)-, c-Kit+, Sca-1+, Flk2-, CD150/SLAMF1+ cells corresponding to putative LT-HSCs (Figure 7A, Figure 13A). Endothelial-specific ablation of Jagged1 in adult animals reduced this fraction substantially (by 90% in individual animals and around 60-70% on average) (Figure 7A). Likewise, the characterization of *Flk1*^{iΔEC} mutants uncovered a similar reduction in the frequency of long-term hematopoietic stem cells (by 60-80% in individual animals and around 75% on average) (Figure 7C; Figure 13A). Since the genetic inactivation of VEGFR2 in ECs also strongly reduced the total number of (hematopoietic) cells in the bone marrow, normalized cell numbers indicate an even more dramatic 5 to 6-fold reduction in the fraction of LT-HSCs (Figure 7C). Hematopoietic cells isolated from endothelial-specific Flk1 mutants also showed reduced multi-lineage colony formation in vitro, while the clonal differentiation of mutant bone marrow cells into single cell types was unaffected or, perhaps as a compensatory measure for maintaining hematopoiesis, even increased (Figure 7D). Finally, competitive transplantation experiments (n=8; analysis at 4 weeks after transplantation into irradiated recipients) revealed that repopulation capacity of stem and progenitor cells from *Flk1*^{iΔEC} mice was reduced by 25% in comparison to GFP-expressing control bone marrow cells (data not shown).

Taken together, these data show that Notch and VEGF signaling in endothelial cells controls angiogenesis of the sinusoidal vasculature, the abundance of Nestin-positive mesenchymal progenitor/stem cells, bone marrow component formation and organization, and thereby the maintenance of long-term hematopoietic stem cells in the adult. Given that the established bone marrow cavity is maintained in the EC-specific mutant models, the changes in hematopoiesis are the consequence of disrupted bone marrow component formation and function.

### References

Adams, G.B., and Scadden, D.T. (2006). The hematopoietic stem cell in its place. Nat. Immunol. 7, 333-337.
Aguirre-Ghiso, J.A. (2007). Models, mechanisms and clinical evidence for cancer dormancy. Nat. Rev. Cancer 7: 834-846.
Armulik, A., Abramsson, A., and Betsholtz, C. (2005). Endothelial/pericyte interactions. Circ. Res. 97, 512-523.
Benedito, R., Roca, C., Sorensen, I., Adams, S., Gossler, A., Fruttiger, M., and Adams, R.H. (2009). The notch ligands DII4 and Jagged1 have opposing effects on angiogenesis. Cell 137, 1124-1135.
Brooker, R., Hozumi, K., and Lewis, J. (2006). Notch ligands with contrasting functions: Jagged1 and Delta1 in the mouse inner ear. Development 133, 1277-1286.
Calvi, L.M., Adams, G.B., Weibrecht, K.W., Weber, J.M., Olson, D.P., Knight, M.C., Martin, R.P., Schipani, E., Divieti, P., Bringhurst, F.R., et al. (2003). Osteoblastic cells regulate the haematopoietic stem cell niche. Nature 425, 841-846.
**Claxton,** S., Kostourou, V., Jadeja, S., Chambon, P., Hodivala-Dilke, K., and Fruttiger, M. (2008). Efficient, inducible Cre-recombinase activation in vascular endothelium. Genesis 46, 74-80. Colmone, A., Amorim, M., Pontier, A.L., Wang, S., Jablonski, E., and Sipkins, D.A. (2008). Leukemic cells create bone marrow niches that disrupt the behavior of normal hematopoietic progenitor cells. Science 322, 1861-1865.
Crisan, M., Yap, S., Casteilla, L., Chen, C.W., Corselli, M., Park, T.S., Andriolo, G., Sun, B., Zheng, B., Zhang, L., et al. (2008). A perivascular origin for mesenchymal stem cells in multiple human organs. Cell Stem Cell 3, 301-313.
Emons, J., Chagin, A.S., Hultenby, K., Zhivotovsky, B., Wit, J.M., Karperien, M., and Savendahl, L. (2009). Epiphyseal fusion in the human growth plate does not involve classical apoptosis. Pediatr. Res. 66, 654-659.
Engin, F., Yao, Z., Yang, T., Zhou, G., Bertin, T., Jiang, M.M., Chen, Y., Wang, L., Zheng, H., Sutton, R.E., et al. (2008). Dimorphic effects of Notch signaling in bone homeostasis. Nat. Med. 14, 299-305.
Erlebacher, A., Filvaroff, E.H., Gitelman, S.E., and Derynck, R. (1995). Toward a molecular understanding of skeletal development. Cell 80, 371-378.
Eshkar-Oren, I., Viukov, S.V., Salameh, S., Krief, S., Oh, C.D., Akiyama, H., Gerber, H.P., Ferrara, N., and Zelzer, E. (2009). The forming limb skeleton serves as a signaling center for limb vasculature patterning via regulation of Vegf. Development 136, 1263-1272.
Gerber, H.P., Vu, T.H., Ryan, A.M., Kowalski, J., Werb, Z., and Ferrara, N. (1999). VEGF couples hypertrophic cartilage remodeling, ossification and angiogenesis during endochondral bone formation. Nat. Med. 5, 623-628.
Gerhardt, H., Golding, M., Fruttiger, M., Ruhrberg, C., Lundkvist, A., Abramsson, A., Jeltsch, M., Mitchell, C., Alitalo, K., Shima, D., et al. (2003). VEGF guides angiogenic sprouting utilizing endothelial tip cell filopodia. J. Cell Biol. 161, 1163-1177.
Gossen, M., and Bujard, H. (1992). Tight control of gene expression in mammalian cells by tetracycline-responsive promoters. Proc. Natl. Acad. Sci. U S A 89, 5547-5551.
Grills, B.L., and Ham, K.N. (1989). Transmission electron microscopy of undecalcified bone. J. Electron Microsc. Tech. 11, 178-179.
Haigh, J.J., Morelli, P.I., Gerhardt, H., Haigh, K., Tsien, J., Damert, A., Miquerol, L., Muhlner, U., Klein, R., Ferrara, N., et al. (2003). Cortical and retinal defects caused by dosage-dependent reductions in VEGF-A paracrine signaling. Dev. Biol. 262, 225-241.
Hellstrom, M., Phng, L.K., Hofmann, J.J., Wallgard, E., Coultas, L., Lindblom, P., Alva, J., Nilsson, A.K., Karlsson, L., Gaiano, N., et al. (2007). DII4 signalling through Notch1 regulates formation of tip cells during angiogenesis. Nature 445, 776-780.
Hilton, M.J., Tu, X., Wu, X., Bai, S., Zhao, H., Kobayashi, T., Kronenberg, H.M., Teitelbaum, S.L., Ross, F.P., Kopan, R., et al. (2008). Notch signaling maintains bone marrow mesenchymal progenitors by suppressing osteoblast differentiation. Nat. Med. 14, 306-314.
Hooper, A.T., Butler, J.M., Nolan, D.J., Kranz, A., lida, K., Kobayashi, M., Kopp, H.G., Shido, K., Petit, I., Yanger, K., et al. (2009). Engraftment and reconstitution of hematopoiesis is dependent on VEGFR2-mediated regeneration of sinusoidal endothelial cells. Cell Stem Cell 4, 263-274.
Kamba, T., Tam, B.Y., Hashizume, H., Haskell, A., Sennino, B., Mancuso, M.R., Norberg, S.M., O'Brien, S.M., Davis, R.B., Gowen, L.C., et al. (2006). VEGF-dependent plasticity of fenestrated capillaries in the normal adult microvasculature. Am. J. Physiol. Heart Circ. Physiol. 290, H560-576.
Kaushansy, K., Lichtman, M.A., Beutler, E., Kipps, T.J., Prchal, J. and Seligsohn, U. (2005). Williams Hematology, Seventh Edition (New York: McGraw-Hill).
Kiel, M.J., Yilmaz, O.H., Iwashita, T., Terhorst, C., and Morrison, S.J. (2005). SLAM family receptors distinguish hematopoietic stem and progenitor cells and reveal endothelial niches for stem cells. Cell 121, 1109-1121.
Li, J.L., Sainson, R.C., Shi, W., Leek, R., Harrington, L.S., Preusser, M., Biswas, S., Turley, H., Heikamp, E., Hainfellner, J.A., et al. (2007). Delta-like 4 Notch ligand regulates tumor angiogenesis, improves tumor vascular function, and promotes tumor growth in vivo. Cancer Res. 67, 11244-11253.
Li, L., and Xie, T. (2005). Stem cell niche: structure and function. Annu. Rev. Cell Dev. Biol. 21, 605-631.
Maes, C., Carmeliet, P., Moermans, K., Stockmans, I., Smets, N., Collen, D., Bouillon, R., and Carmeliet, G. (2002). Impaired angiogenesis and endochondral bone formation in mice lacking the vascular endothelial growth factor isoforms VEGF164 and VEGF188. Mech. Dev. 111, 61-73.
Maes, C., Goossens, S., Bartunkova, S., Drogat, B., Coenegrachts, L., Stockmans, I., Moermans, K., Nyabi, O., Haigh, K., Naessens, M., et al. (2010). Increased skeletal VEGF enhances beta-catenin activity and results in excessively ossified bones. EMBO J. 29, 424-441.
Mendez-Ferrer, S., Michurina, T.V., Ferraro, F., Mazloom, A.R., Macarthur, B.D., Lira, S.A., Scadden, D.T., Ma'ayan, A., Enikolopov, G.N., and Frenette, P.S. (2010). Mesenchymal and haematopoietic stem cells form a unique bone marrow niche. Nature 466, 829-834. Moore, K.A., and Lemischka, I.R. (2006). Stem cells and their niches. Science 311, 1880-1885.
Morrison, S.J., and Spradling, A.C. (2008). Stem cells and niches: mechanisms that promote stem cell maintenance throughout life. Cell 132, 598-611.
Muzumdar, M.D., Tasic, B., Miyamichi, K., Li, L., and Luo, L. (2007). A global double-fluorescent Cre reporter mouse. Genesis 45, 593-605.
Sacchetti, B., Funari, A., Michienzi, S., Di Cesare, S., Piersanti, S., Saggio, I., Tagliafico, E., Ferrari, S., Robey, P.G., Riminucci, M., et al. (2007). Self-renewing osteoprogenitors in bone marrow sinusoids can organize a hematopoietic microenvironment. Cell 131, 324-336.
Scadden, D.T. (2006). The stem-cell niche as an entity of action. Nature 441, 1075-1079.
Siekmann, A.F., and Lawson, N.D. (2007). Notch signalling limits angiogenic cell behaviour in developing zebrafish arteries. Nature 445, 781-784.
Sugiyama, T., Kohara, H., Noda, M., and Nagasawa, T. (2006). Maintenance of the hematopoietic stem cell pool by CXCL12-CXCR4 chemokine signaling in bone marrow stromal cell niches. Immunity 25, 977-988.
Sun, J.F., Phung, T., Shiojima, I., Felske, T., Upalakalin, J.N., Feng, D., Kornaga, T., Dor, T., Dvorak, A.M., Walsh, K., et al. (2005). Microvascular patterning is controlled by fine-tuning the Akt signal. Proc. Natl. Acad. Sci. U S A 102, 128-133.
Suzuki, H., Amizuka, N., Oda, K., Li, M., Yoshie, H., Ohshima, H., Noda, M., and Maeda, T. (2005). Histological evidence of the altered distribution of osteocytes and bone matrix synthesis in klotho-deficient mice. Arch. Histol. Cytol. 68, 371-381.
Trindade, A., Kumar, S.R., Scehnet, J.S., Lopes-da-Costa, L., Becker, J., Jiang, W., Liu, R., Gill, P.S., and Duarte, A. (2008). Overexpression of delta-like 4 induces arterialization and attenuates vessel formation in developing mouse embryos. Blood 112, 1720-1729.
Trump, A. et al. (2010). Awakening dormant haematopoeietic stem cells. Nat. Rev. Immunol. 10, 201-209.
Voog, J., D'Alterio, C., and Jones, D.L. (2008). Multipotent somatic stem cells contribute to the stem cell niche in the Drosophila testis. Nature 454, 1132-1136.
Wang, Y., Nakayama, M., Pitulescu, M.E., Schmidt, T.S., Bochenek, M.L., Sakakibara, A., Adams, S., Davy, A., Deutsch, U., Luthi, U., et al. (2010). Ephrin-B2 controls VEGF-induced angiogenesis and lymphangiogenesis. Nature 465, 483-486.
Williams, C.K., Li, J.L., Murga, M., Harris, A.L., and Tosato, G. (2006). Up-regulation of the Notch ligand Delta-like 4 inhibits VEGF-induced endothelial cell function. Blood 107, 931-939.
Wilson, A. et al., (2008). Hematopoietic stem cells reversibly switch from dormancy to self-renewal during homeostasis and repair. Cell 135, 118-1129.
Zeuschner, D., Mildner, K., Zaehres, H., and Scholer, H.R. (2010). Induced pluripotent stem cells at nanoscale. Stem Cells Dev. 19, 615-620.
Zhang, J., Niu, C., Ye, L., Huang, H., He, X., Tong, W.G., Ross, J., Haug, J., Johnson, T., Feng, J.Q., et al. (2003). Identification of the haematopoietic stem cell niche and control of the niche size. Nature 425, 836-841.

## Claims

1. A bone marrow component comprising at least one capillary embedded in a bone culture wherein the capillary comprises
(i) an inner cell layer of endothelial cells surrounding an inner lumen that is connected to the vasculature; and
(ii) an outer cell layer comprising mesenchymal cells, osteoblasts, and osteoclasts; and wherein at least part of the distal end of capillary is dilated compared to the proximal end and at least part of the dilated end has an outer cell layer that is physically separated from the inner cell layer.

2. The bone marrow component of claim 1, further comprising (iii) at least one hematopoietic cell, wherein the hematopoietic cell is located between the inner cell layer and the outer cell layer at the dilated distal end of the capillary.

3. The bone marrow component of claim 1 or 2 wherein the bone culture comprises non-native materials.

4. A ligand selected from Jagged1, DLL4, or VEGF for use in modulating the bone marrow component of claim 1 in a subject.

5. A method of monitoring bone marrow status of a subject comprising detecting at least one bone marrow component of claim 1 within a bone marrow sample provided from a subject.

6. A method of identifying
(i) a compound that modulates bone marrow homeostasis, or
(ii) a compound that modulates angiogenesis,
comprising detecting at least one bone marrow component of claim 1 within a bone marrow sample provided from a subject that has previously been administered a compound, wherein a change in the morphology of the bone marrow component indicates that the compound modulates bone marrow homeostasis or angiogenesis.

7. A method of identifying
(i) a compound that inhibits cancer, or
(ii) whether a compound causes adverse side effects on bone marrow homeostasis or hematopoiesis,
comprising detecting whether a change in numbers or morphology of bone marrow components of claim 1 has occurred within a bone marrow sample provided from a subject that has previously been administered a compound, wherein a reduction of bone marrow components or change in bone marrow component morphology indicates that the compound inhibits cancer or causes adverse side effects on bone marrow homeostasis or hematopoiesis.

8. The method of claim 7 wherein detecting a change in bone marrow component morphology, bone marrow homeostasis, or hematopoiesis comprises detecting whether a reduction in HSC or LT-HSC associated with the bone marrow component, a reduction in sinusoidal endothelial cell growth, or a reduction of the dilation of sinusoidal capillaries has occurred.

9. A method of identifying a compound that promotes trabecular bone formation comprising detecting at least one bone marrow component of claim 1 within a bone marrow sample provided from a subject from a subject that has previously been administered a compound, wherein an increase in numbers of bone marrow components indicates that the compound promotes trabecular bone formation.

10. The method of any one of claims 5 to 9 wherein detecting bone marrow components or detecting a change in bone marrow component morphology comprises assaying bone marrow component development or bone marrow component homeostasis.

11. The method of claim 10, wherein assaying bone marrow component development or bone marrow component homeostasis comprises at least one of detecting the frequency of bone marrow components, detecting the number bone marrow components, and detecting the frequency or number of capillaries having at least part of an inner endothelial cell layer detached from the outer mesenchymal cell layer.

12. The method of claim 10, wherein assaying bone marrow component development or bone marrow component homeostasis comprises at least one of detecting the frequency of Nestin+ mesenchymal cells, detecting the number of Nestin+ mesenchymal cells, detecting the frequency of F-actin protrusion-containing mesenchymal cells or osteoblastic cells, and detecting the number of F-actin+ mesenchymal cells or osteoblastic cells.

13. The method of claim 10, wherein assaying bone marrow component development or bone marrow component homeostasis comprises at least one of detecting the frequency of hematopoietic stem cells (HSC) associated with bone marrow components, detecting the number of HSC associated with bone marrow components, detecting the frequency of long-term hematopoietic stem cells (LT-HSC) associated with bone marrow components, and detecting the number of LT-HSC associated with bone marrow components.

14. The method of any one of claims 5 to 10 wherein the subject is a transgenic mouse

15. The method of claim 14, wherein the transgenic mouse is the offspring of the combination of an endothelial-specific, tamoxifen-inducible *Cdh5*(PAC)-*Cre*ERT2 transgenic mouse and a m*T*/mG Cre reporter mouse.

16. The method of any one of claims 5 to 10 wherein the cancer is a non-solid cancer.

17. The method of claim 16, wherein the non-solid cancer is leukemia.

18. A method of identifying whether a compound modulates the conversion of a dormant hematopoietic stem cell to an active hematopoietic stem cell comprising detecting a bone marrow component of claim 1 within a bone marrow sample provided from a subject that has previously been administered a compound, wherein an increase in the number of active hematopoietic stem cells indicates that the compound modulates the conversion of a dormant hematopoietic stem cell to an active hematopoietic stem cell.
